# EUROPEAN PATENT SPECIFICATION

(11) **EP 1 760 095 B1**
(45) Date of publication and mention of the grant of the patent: **31.03.2010**
(21) Application number: 06017910.8
(22) Date of filing: 28.08.2006
(51) Int. Cl.: C08F 2/24, C08F 2/26

(54) **Emulsifier for emulsion polymerization, production method of polymer emulsion and polymer emulsion**
Emulgator für die Emulsionspolymerisation, Herstellungsmethode für Polymeremulsionen und Polymeremulsionen
Émulsifiant pour la polymérisation en émulsion, procédé de production d'émulsions de polymères et émulsions de polymères

(30) Priority: 29.08.2005 JP 2005248460; 29.08.2005 JP 2005248477; 29.09.2005 JP 2005284056; 02.11.2005 JP 2005319883; 10.11.2005 JP 2005325896
(43) Date of publication of application: 07.03.2007
(73) Proprietor: Dai-Ichi Kogyo Seiyaku Co., Ltd., Kyoto-shi, Kyoto 600-8873 (JP)
(72) Inventor: Hashimoto, Masayuki, Nishinomiya-shi, Hyogo 663-8101 (JP); Hada, Yasunobu, Ukyo-ku, Kyoto-shi Kyoto 615-0814 (JP); Matsubara, Toyoharu, Osaka-shi, Osaka 547-0013 (JP); Mukai, Tetsuya, Minami-ku, Kyoto-shi, Kyoto 601-8023 (JP)
(74) Representative: Strehl Schübel-Hopf & Partner

(56) References cited:
- EP-A- 0 244 841
- EP-A- 0 450 451

## Description

### BACKGROUND OF THE INVENTION

### 1. Field of the Invention

The present invention relates to an emulsifier for emulsion polymerization, used in emulsion polymerizing, a production method of a polymer emulsion using the emulsifier for emulsion polymerization, and a polymer emulsion obtained by the production method.

### 2. Background Art

Conventionally, as a nonionic emulsifier for emulsion polymerization, a polyoxyalkylene alkylphenyl ether comprising nonyl phenol or octyl phenol having an alkylene oxide added thereto, and a polyoxyalkylene alkylether comprising a higher alcohol having an alkylene oxide added thereto are used alone or in combination.

Further, as an emulsifier for emulsion polymerization, an anionic surfactant such as dodecylbenzene sulfonic acid salts, alkylsulfuric acid ester salts, alkylsulfosuccinic acid ester salts and polyoxyalkylene alkyl (aryl) ethersulphates acid ester salts are used alone or in combination with a nonionic surfactant such as polyoxyalkylene alkyl(aryl)ether and polyoxyethylene polyoxypropylene block copolymer.

However, the above emulsifier for emulsion polymerization cannot always sufficiently be satisfactory in stability of a polymer emulsion, or properties of a polymer film obtained from the emulsion, and many problems to be solved remain. For example, there are the problems on polymerization stability of an emulsion; mechanical stability, chemical stability, freeze-thaw stability, pigment miscibility, storage stability of an emulsion obtained.

Further, when a polymer film is prepared from an emulsion, an emulsifier used remains in the polymer film in a free state, and this gives rise to the problems of poor water resistance and adhesion of a film. Further, when an emulsion is destroyed with the means such as salting-out or acid dipping to take out a polymer, a large amount of an emulsifier is contained in a wastewater, and this induces environmental pollution such as river contamination. Therefore, a large amount of labor is required for removal treatment of the emulsifier.

To improve the problems of the conventional emulsifier for emulsion polymerization from such a standpoint, a reactive emulsifier having a copolymerizable unsaturated group as a reactive group and a polyoxyalkylene chain as a hydrophilic group is proposed in, for example, JP-A-8-41112, JP-A-4-50204, JP-A-63-319035 and JP-A-62-104802, and emulsion polymerization is attempted on various monomers.

Further, an anionic reactive emulsifier is disclosed in JP-A-1-99638, JP-A-58-203960, and a nonionic polymerizable surfactant is described in JP-A-2003-268021, JP-A-4-50204, JP-A-63-54927. Thus, emulsion polymerization is attempted on various monomers.

Emulsions using those reactive emulsifiers as an emulsion polymerization agent have good stability at the time of polymerization, and polymer films obtained from those emulsions show excellent performances in water resistance, adhesion, heat resistance and weather resistance.

However, the problems of a reactive emulsifier derived from such an alkylene oxide are that an unreacted alkylene oxide remains in a product, and substances having high carcinogenicity and irritating properties generate as a by-product. For example, it is known that harmful dioxane generates at the time of synthesis, and harmful aldehydes generate by oxidative decomposition of an alkylene oxide chain.
In recent years that sick house syndrome or VOC (volatile organic compound) problem receives a lot of publicity, it is not preferable to use an emulsifier for emulsion polymerization containing aldehydes in the production of an emulsion.

EP 0 45 0451 discloses aqueous emulsions of polymers prepared with crosslinkable non-ionic surfactants free of oxy-ethylene units.

### SUMMARY OF THE INVENTION

The present invention has been made in view of the above circumstance, and its object is to provide a reactive emulsifier for emulsion polymerization that does not have a polyoxyalkylene chain as a hydrophilic group, has good stability at the time of emulsion polymerization, and gives a polymer and a polymer film, having greatly improved various properties such as water resistance, adhesion, heat resistance and weather resistance.

As a result of keen investigations to overcome the above problems, the present inventors have found that an emulsifier for emulsion polymerization having an allyl group or a methallyl group as a copolymerizable unsaturated group, and having a hydrophilic group moiety comprising a polyglycerin, or a polyglycerin and an anionic hydrophilic group is suitable, and have reached the present invention.

The present invention provides the following.
(1) An emulsifier for emulsion polymerization, comprising a compound represented by the following general formula (1): 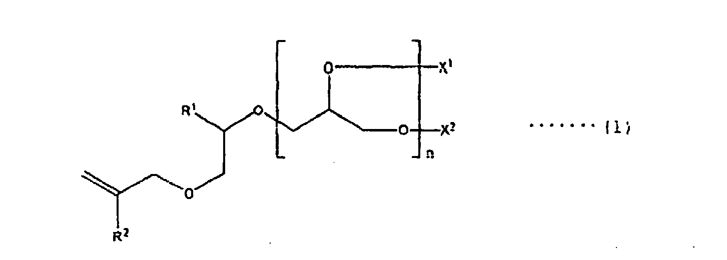 wherein R¹ represents a substituent represented by R or -CH₂-O-R wherein R is selected from the group consisting of decyl, secondary decyl, undecyl, secondary undecyl, dodecyl, secondary dodecyl, tridecyl, isotridecyl, secondary tridecyl, tetradecyl, secondary tetradecyl, hexadecyl, secondary hexadecyl, 2-butyloctyl, 2-butyldecyl, 2-hexyloctyl, 2-hexyldecyl and styrenated phenyl, and additionally -O-R may be a residue of an alcohol selected from the group consisting of lauryl alcohol, myristyl alcohol, cetyl alcohol, isodecanol, isoundecanol, isododecanol, isotridecanol, 2-propyl-1-octanol, 2-propyl-1-heptanol, 4-methyl-2-propyl-1-hexanol and 2-propyl-5-methyl-1-hexanol; R² represents a hydrogen atom or a methyl group; n represents from 1 to 200; X¹ and X² each represents a hydrogen atom, a hydrocarbon group or an anionic hydrophilic group.
(2) The emulsifier for emulsion polymerization as defined in (1), wherein the general formula (1) has at least one of anionic hydrophilic groups represented by the following general formulae (2) to (6) as the anionic hydrophilic group: 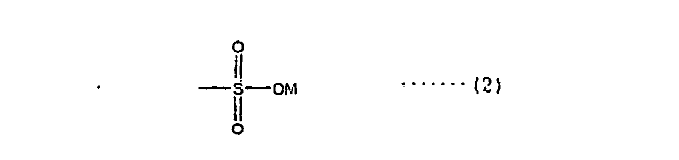 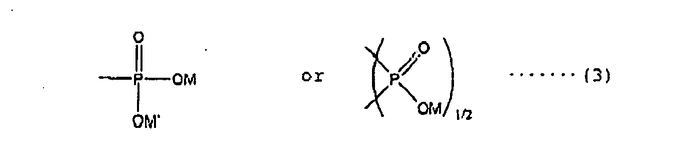 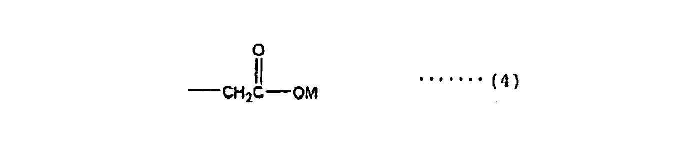 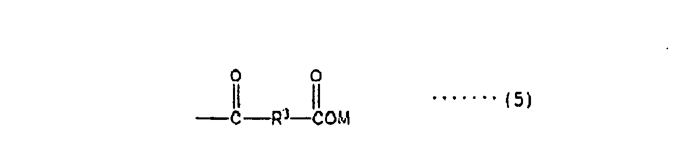 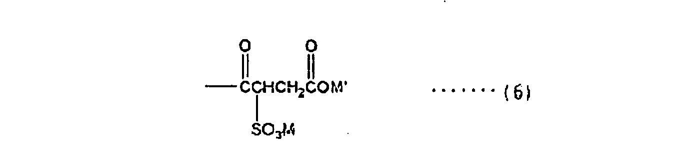 wherein R³ represents a residue that a carboxyl group is eliminated from a dibasic acid; M and M' which may be the same or different each represents a hydrogen atom, a metallic atom, ammonium or a hydrocarbon group, wherein, if only M is present, M is a metallic atom or ammonium, and, if both M and M' are present, at least one of M and M' is a metallic atom or ammonium.
(3) An emulsifier for emulsion polymerization, comprising a mixture of the compound as defined in (1) or (2) with at least one surfactant selected from the group consisting of nonionic surfactants, anionic surfactants, and cationic surfactants, other than the compound.
(4) A production method of a polymer emulsion, which comprises polymerizing monomers using the emulsifier for emulsion polymerization as defined in any one of (1) to (3) in an amount of from 0.1 to 20% by weight based on the weight of the entire monomers in an aqueous medium, or adding the emulsifier for emulsion polymerization to a polymer after polymerization of the monomers.
(5) A polymer emulsion obtained by the production method of a polymer emulsion as defiend in (4).

### BRIEF DESCRIPTION OF THE DRAWING

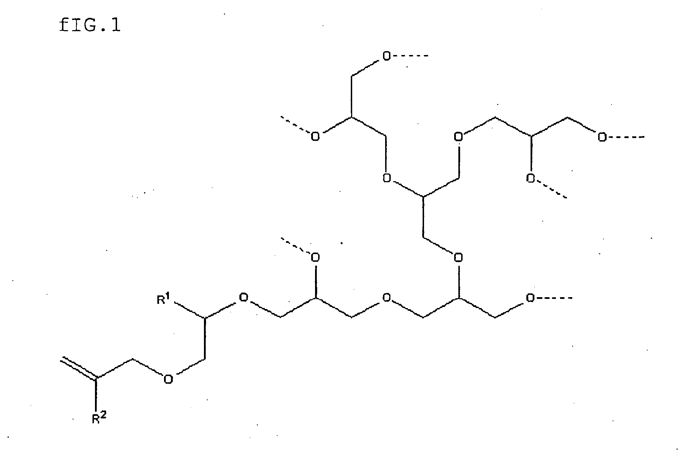

Fig. 1 is a schematic structural view showing one example of a polyglycerin moiety connected in a dendritic form.

### DETAILED DESCRIPTION OF THE INVENTION

The embodiment of the emulsifier for emulsion polymerization of the present invention is described below.

The emulsifier for emulsion polymerization of the present invention contains a compound represented by the following general formula (1),

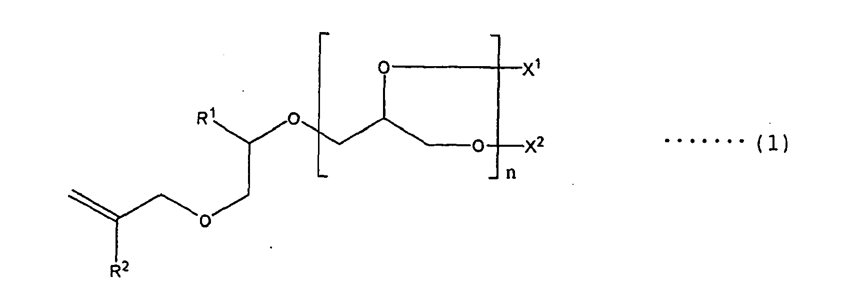

In the above general formula (1), R¹ represents a substituent represented by R or -CH₂-O-R wherein R is selected from the group consisting of decyl, secondary decyl, undecyl, secondary undecyl, dodecyl, secondary dodecyl, tridecyl, isotridecyl, secondary tridecyl, tetradecyl, secondary tetradecyl, hexadecyl, secondary hexadecyl, 2-butyloctyl, 2-butyldecyl, 2-hexyloctyl, 2-hexyldecyl, and styrenated phenyl, and additionally -O-R may be a residue of an alcohol selected from the group consisting of lauryl alcohol, myristyl alcohol, cetyl alcohol, isodecanol, isoundecanol, isododecanol, isodecanol, 2-propyl-1-octanol, 2-propyl-1-heptanol, 4-methyl-2-propyl-1-hexanol and 2-propyl-5-methyl-1-hexanol.

Those alcohols can be used as mixtures of two or more thereof.

In the general formula (1), R² is a hydrogen atom or a methyl group.

In the general formula (1), X¹ and X² are a hydrogen atom, a hydrocarbon group or an anionic hydrophilic group. Examples of the hydrocarbon group include the hydrocarbon groups described above.

Examples of the anionic hydrophilic group include a sulfate group, (formula 2), a phosphate group (formula 3), a carboxylate group (formula 4 or 5) and sulfosuccinate group (formula 6), represented by the following general formulae (2) to (6).

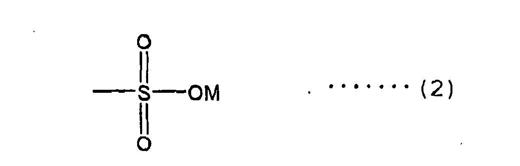

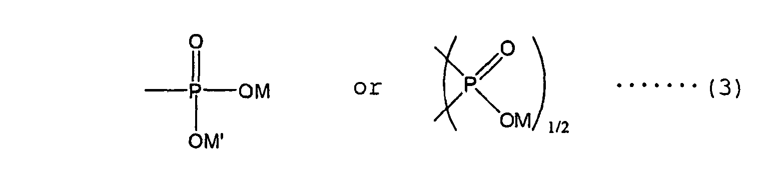

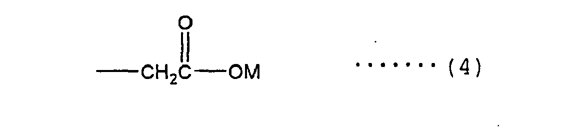

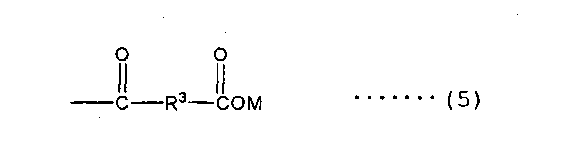

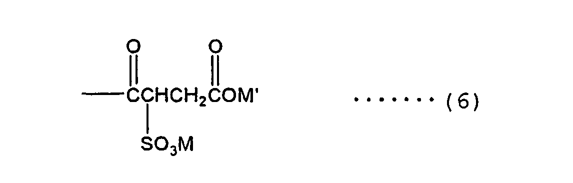

In the formulae (2) to (6), R³ represents a residue that a carboxyl group is eliminated from a dibasic acid. M and M' each represents a hydrogen atom, a metallic atom, ammonium or a hydrocarbon group, and M and M' may be the same or different, wherein, if only M is present, M is a metallic atom or ammonium, and, if both M and M' are present, at least one of M and M' is a metallic atom or ammonium.

Examples of the dibasic acid for constituting R³ include saturated aliphatic dicarboxylic acids such as oxalic acid, malonic acid, succinic acid, glutaric acid, adipic acid, pimelic acid, suberic acid, azelaic acid, cebasic acid, undecanedionic acid, dodecanedionic acid, tridecanedionic acid and tetradecanedionic acid; saturated alicyclic dicarboxylic acids such as cyclopentanedicarboxylic acid, hexahydrophthalic acid and methylhexahydrophthalic acid; aromatic dicarboxylic acids such as phthalic acid, isophthalic acid, terephthalic acid, tolylenedicarboxylic acid and xylylenedicarboxylic acid; unsaturated aliphatic dicarboxylic acids such as maleic acid, fumaric acid, itaconic acid, citraconic acid and mesaconic acid; and unsaturated alicyclic dicarboxylic acids such as tetrahydrophthalic acid, methyltetrahydrophthalic acid, nadic acid (endomethylenetetrahydrophthalic acid), methylnadic acid, methylbutenyltetrahydrophthalic acid and methylpentenyltetrahydrophthalic acid.

M and M' each represents a hydrogen atom, a metallic atom, ammonium or a hydrocarbon group. Examples of the metallic atom include alkali metal atoms such as lithium, sodium and potassium; and alkaline earth metal atoms (alkaline earth metal atom is generally divalent, and therefore 1/2) such as magnesium and calcium. Examples of the ammonium include ammonia, methylamine, dimethylamine, ethylamine, diethylamine, (iso)propylamine, di(iso)propylamine, monoethanolamine, N-methyl monoethanolamine, N-ethylmonoethanolamine, diethanolamine, triethanolamine, monopropanolamine, dipropanolamine, tripropanolamine, 2-amino-2-methyl-1,3- propanediol, aminoethylethanolamine, N,N,N',N'-tetrakis (2- hydroxypropyl)ethylenediamine. Examples of the hydrocarbon groups include the above-described alkyl group, alkenyl group, aryl group, cycloalkyl group and cycloalkenyl group. M and M' may be the same or different, and may contain at least two of the above-described examples.

The emulsifier for emulsion polymerization of the present invention may contain two or more of the above-described anionic hydrophilic groups as X¹ and X² in the general formula (1).

In the general formula (1), n is from 1 to 200, and preferably from 1 to 60.

The polyglycerin moiety in the general formula (1) may be connected linearly or in a dendritic form as shown in Fig. 1.

The emulsifier for emulsion polymerization of the present invention is advantageous that in the case of not containing an anionic hydrophilic group, a molar ratio of the hydrocarbon group moiety and the polyglycerin moiety is from 1:1 to 5:1, and in the case of containing an anionic hydrophilic group, a molar ratio of the hydrocarbon group moiety and the polyglycerin moiety is from 1:1 to 5:1, and a molar ratio of the anionic hydrophilic group moiety and the polyglycerin moiety is from 1:1 to 5:1.

### Synthesis method of emulsifier for emulsion polymerization:

Reaction conditions for obtaining the emulsifier for emulsion polymerization of the present invention are not particularly limited. For example, when R¹ is R, α-olefin epoxide and allyl alcohol or methallyl alcohol, and when R¹ is -CH₂-O-R, a higher alcohol and an allylglycidyl ether; or an alkylglycidyl ether derived from a higher alcohol and epichlorohydrin, and allyl alcohol, are reacted in the presence of a catalyst, and the polyglycerin moiety is introduced by the conventional method. In the case of introducing the anionic hydrophilic group, various anionic groups are introduced into the polyglycerin moiety of the reaction composition obtained, thereby obtaining the emulsifier for emulsion polymerization of the present invention. According to need, purification of the emulsifier obtained by the conventional method may be conducted.

A method of introducing the polyglycerin into a hydrophobic group moiety having a reactive group can be conducted by applying the conventional production method of a polyglycerin alkyl ether. The production method of the polyglycerin alkyl ether can be conducted by the methods described in, for example, JP-A-2001-114720, JP-A-2000-38365, JP-A-9-188755 and JP-A-6-293688.

Further, introduction of an anionic group into the polyglycerin moiety can be conduced using the conventional method. For example, introduction of an anionic hydrophilic group of the general formula (2) can be achieved by sulfate esterification using sulfamic acid, chlorosulfonic acid, sulfuric anhydride or sulfuric acid. Introduction of the anionic hydrophilic group of the general formula (3) can be achieved by phosphate esterification using diphosphorus pentoxide or polyphosphoric acid. Introduction of the anionic hydrophilic group of the general formula (4) can be achieved by ether carboxylation using a monohalogen lower carboxylic acid (monochloroacetic acid or monobromopropionic acid). Introduction of the anionic hydrophilic group of the general formula (5) can be achieved by ester carboxylation using a dibasic acid (anhydride is preferable). Introduction of the anionic hydrophilic group of the general formula (6) can be achieved by ester carboxylation with maleic anhydride, and then sulfonation with sodium sulfite.

### Monomer for emulsion polymerization:

Monomers applicable to emulsion polymerization using the emulsifier for emulsion polymerization of the present invention can include various monomers, and examples thereof include acrylic monomers such as acrylic acid, methyl acrylate, butyl acrylate, 2-ethylhexyl acrylate, methyl methacrylate, acrylonitrile, acrylamide and hydroxyacrylic acid ester; aromatic monomers such as styrene and divinylbenzene; vinyl ester monomers such as vinyl acetate; halogenated olefin monomers such as vinyl chloride and vinylidene chloride; conjugated diolefin monomers such as butadiene, isoprene and chloroprene; ethylene, maleic anhydride and methyl maleate.
The emulsifier for emulsion polymerization of the present invention can be utilized to emulsion polymerization or suspension polymerization of at least one of the above monomers.

### Polymerization condition:

A polymerization initiator used in emulsion polymerization reaction using the emulsifier for emulsion polymerization of the present invention may be conventional compounds, and for example, hydrogen peroxide, potassium persulfate, azobisisobutyronitrile and benzoyl peroxide can be utilized. As a polymerization accelerator, sodium hydrogensulfite and ferrous ammonium sulfate can be used. As a chain transfer agent, mercaptans such as α-methylstyrene dimer, n-butylmercaptan and t-dodecylmercaptan; halogenated hydrocarbons such as carbon tetrachloride and carbon tetrabromide may be used.

The amount of the emulsifier for emulsion polymerization of the present invention used is generally from 0.1 to 20.0% by weight, and preferably from 0.2 to 10.0% by weight, based on the weight of the entire monomers.

The emulsifier for emulsion polymerization of the present invention can well complete the emulsion polymerization by it alone, but may be used in combination with other nonionic surfactant, anionic surfactant and cationic surfactant. The combined use can improve polymerization stability at the time of emulsion polymerization, and further can improve treatment characteristics in a post-treatment. The treatment characteristics means coating process or dipping process.

The other nonionic surfactant, anionic surfactant and cationic surfactant are not particularly limited. Examples of the nonionic surfactant include polyoxyalkylene alkylphenyl ether, polyoxyalkylene alkyl ether, alkyl polyglucoside, polyglycerin alkyl ether, polyglycerin fatty acid ester, polyoxyalkylene fatty acid ester and sorbitan fatty acid ester. Examples of the anionic surfactant include fatty acid soap, rosinic acid soap, alkylsulfonic acid salt, alkylarylsulfonic acid salt, alkylsulfosuccinic acid salt, polyoxyethylene alkylsulfuric acid and polyoxyethylene arylsulfuric acid salt. Examples of the cationic surfactant include stearyl trimethyl ammonium, cetyl trimethyl ammonium and lauryl trimethyl ammonium. The amount of those other surfactants used is preferably from 0.5 to 100 parts by weight, more preferably from 5 to 60 parts by weight, and most preferably from 10 to 30 parts by weight, per 100 parts by weight of the emulsifier for emulsion polymerization of the present invention.

The conventional protective colloidal agent can be used together for the purpose of improving polymerization stability at the time of emulsion polymerization. Examples of the protective colloidal agent that can be used together include a completely saponified polyvinyl alcohol (PVA), partially saponified PVA, hydroxyethyl cellulose, carboxymethyl cellulose, methyl cellulose, polyacrylic acid and gum arabic.

Other method of using the emulsifier for emulsion polymerization of the present invention is that the emulsifier is added to a polymer after completion of the polymerization in order to improve stability of the polymer emulsion.

### Action and other:

The emulsifier for emulsion polymerization of the present invention has an allyl group or a methallyl group, which has a copolymerizable double bond, in a hydrophobic group moiety in the molecule, has excellent copolymerizability to a polymerizable monomer, particularly a vinyl monomer, and is liable to be incorporated into a polymer composition. For this reason, the amount of the emulsifier present as a copolymerizable reactive emulsifier in a free state in a polymer film obtained from the polymer emulsion is greatly reduced, thereby exhibiting extremely excellent effect on water resistance, adhesion, heat resistance and weather resistance of the film. Additionally, foaming, mechanical stability of the polymer emulsion are remarkably improved.

Further, a polymer emulsion containing a greatly reduced amount of harmful substances such as dioxane and aldehydes can be obtained.

The polymer emulsion obtained by adding the emulsifier for emulsion polymerization of the present invention can be applied to woods, metals, papers, fabrics and concretes as, for example, an adhesive, a covering material or an impregnating reinforcement. Further, the polymer taken out of the emulsion or latex can be used as a modifier of resins, rubbers, and polymers.

### Examples

The present invention is described in more detail by reference to the following Examples and Comparative Examples, but it should be understood that the invention is not construed as being limited thereto. Unless otherwise indicated, "part" is by weight.

### First Embodiment

### PRODUCTION EXMAPLE 1

87 parts of allyl alcohol and a borotrifluoride ether complex as a catalyst were placed in a reactor equipped with a stirrer, a nitrogen introduction pipe and a thermometer, and 196 parts of α-olefin epoxide having 12, 14 carbon atoms (AOE X24, a product of Dicel Chemical Industries, Ltd.) was added dropwise thereto to conduct reaction at 80°C for 5 hours under stirring. Temperature was elevated to 120°C, and excess allyl alcohol was removed by reduced pressure. Potassium hydroxide as a catalyst was added to the reaction composition obtained, and 370 parts of glycidol was added dropwise at 120°C over 1 hour, followed by stirring for 2 hours. Finally, water was added to a polyglycerin adduct obtained by the reaction, and the resulting mixture was passed through a cation exchange resin and an anion exchange resin to desalt, followed by dehydration under reduced pressure, thereby obtaining an emulsifier for emulsion polymerization 1A of the present invention.

### PRODUCTION EXAMPLE 2

87 parts of allyl alcohol and potassium hydroxide as a catalyst were placed in a reactor equipped with a stirrer, a nitrogen introduction pipe and a thermometer, and 196 parts of α-olefin epoxide having 12, 14 carbon atoms (Epocizer M-24, a product of Dainippon Ink and Chemicals, Incorporated) was added dropwise thereto to conduct reaction at 80°C for 5 hours under stirring. Temperature was elevated to 120°C, and excess allyl alcohol was removed by reduced pressure. 740 parts of glycidol was added dropwise to the reaction composition obtained at 120°C over 1 hour, followed by stirring for 2 hours. Finally, water was added to a polyglycerin adduct obtained by the reaction, and the resulting mixture was passed through a cation exchange resin and an anion exchange resin to desalt, followed by dehydration under reduced pressure, thereby obtaining an emulsifier for emulsion polymerization 1B of the present invention.

### PRODUCTION EXAMPLE 3

An emulsifier for emulsion polymerization 1C of the present invention was obtained by conducting the reaction and purification under the same conditions as in Production Example 2, except for using 1,480 parts of glycidol.

### PRODUCTION EXAMPLE 4

108 parts of methallyl alcohol and potassium hydroxide as a catalyst were placed in a reactor equipped with a stirrer, a nitrogen introduction pipe and a thermometer, and 212 parts of α-olefin epoxide having 14 carbon atoms (Vikolox 14, a product of ARKEMA Inc.) was added dropwise thereto to conduct reaction at 80°C for 5 hours under stirring. Temperature was elevated to 120°C, and excess methallyl alcohol was removed by reduced pressure. 2,960 parts of glycidol was added dropwise to the reaction composition obtained at 120°C over 1 hour, followed by stirring for 2 hours. Finally, sulfuric acid was added to a polyglycerin adduct obtained by the reaction to neutralize, followed by dehydration. Precipitates were filtered off to obtain an emulsifier for emulsion polymerization 1D of the present invention.

### PRODUCTION EXAMPLE 5

An emulsifier for emulsion polymerization 1E of the present invention was obtained by conducting the reaction and purification under the same conditions as in Production Example 2, except for using 252 parts of α-olefin epoxide having 16, 18 carbon atoms (Epocizer M-68, a product of Dainippon Ink and Chemicals, Incorporated) and 4,440 parts of glycidol.

### PRODUCTION EXAMPLE 6

An emulsifier for emulsion polymerization 1F of the present invention was obtained by conducting the reaction and neutralization under the same conditions as in Production Example 4, except for using 252 parts of α-olefin epoxide having 16, 18 carbon atoms (AOE X68, a product of Dicel Chemical Industries, Ltd.) and 7,400 parts of glycidol.

### PRODUCTION EXAMPLE 7

An emulsifier for emulsion polymerization 1G of the present invention was obtained by conducting the reaction and purification under the same conditions as in Production Example 1, except for using 198 parts of branched α-olefin epoxide having 12 to 14 carbon atoms and 1,480 parts of glycidol.

### PRODUCTION EXAMPLE 8

An emulsifier for emulsion polymerization 1H of the present invention was obtained by conducting the reaction and neutralization under the same conditions as in Production Example 4, except for using 240 parts of branched α-olefin epoxide having 16 carbon atoms and 2,960 parts of glycidol.

### EXAMPLES AND COMPARATIVE EXAMPLES

Emulsifiers for emulsion polymerization used in the Examples and Comparative Examples are shown in Table 1 below. Comparisons 1A to 1F are the conventional emulsifiers for emulsion polymerization, having added thereto the respective ethylene oxide having the chemical structure as shown in Table 1 below.

**TABLE 1**

| | | R^{*1} | R² | Number of glycerin unit |
|---|---|---|---|---|
| Invention | 1A | C10,12 alkyl group | Hydrogen atom | 5 |
| | 1B | C10,12 alkyl group | Hydrogen atom | 10 |
| | 1C | C10,12 alkyl group | Hydrogen atom | 20 |
| | 1D | C12 alkyl group | Methyl group | 40 |
| | 1E | C14,16 alkyl group | Hydrogen atom | 60 |
| | 1F | C14,16 alkyl group | Methyl group | 100 |
| | 1G | Branched C10-12 alkyl group | Hydrogen atom | 20 |
| | 1H | Branched C14 alkyl Group | Methyl group | 40 |
| Comparison | | Structure (EO represents ethylene oxide) | | |
| | 1A | 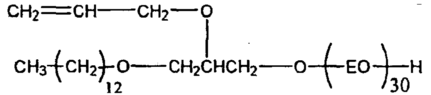 | | |
| | 1B | 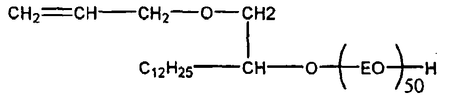 | | |
| | 1C | 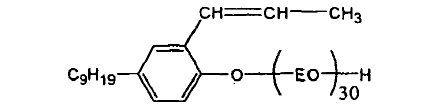 | | |
| | 1D 1E | 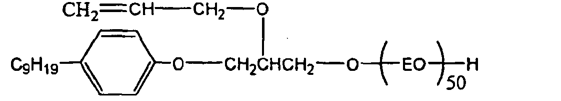 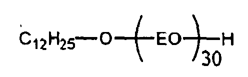 | | |
| | 1F | 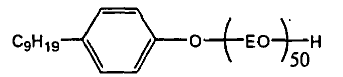 | | |

| | | | | |
|---|---|---|---|---|
| *1 : R¹=R | | | | |

### USE EXAMPLE 1

100 parts of butyl acrylate, 100 parts of styrene, 290 parts of ion-exchanged water and 10 parts of an emulsifier for emulsion polymerization were mixed to prepare a mixed monomer suspension, and dissolved oxygen was removed with nitrogen gas. 100 parts of the mixed monomer suspension was placed in a reactor equipped with a stirrer, a reflux condenser, a thermometer and a dropping funnel. Temperature was elevated to 80°C, 0.5 part of potassium persulfate was added to conduct preceding polymerization. 400 parts of the remaining mixed monomer suspension was added dropwise to the reactor over 3 hours from 10 minutes after polymerization initiation to conduct polymerization. Continuously, the reaction mixture was aged at the polymerization temperature for 2 hours, and cooled to obtain a polymer emulsion.

The emulsifier for emulsion polymerization used is shown in Table 2 below. 10% by weight in the emulsifier for emulsion polymerization was laurylsulfuric acid ester sodium salt as an anionic emulsifier used in combination.

Content of dioxane and formaldehyde in the emulsifier for emulsion polymerization used; polymerization stability, mechanical stability, particle size and foaming property of the polymer emulsion obtained; and luster of the film were evaluated. The evaluation methods are as follows. The results obtained are shown in Table 2 below. Content of dioxane and formaldehyde:
Dioxane contained in the emulsifier for emulsion polymerization was quantified with GC, and formaldehyde was quantified with an absorption spectroscopy using an acetyl acetone method. Evaluation criteria for the content of each of dioxane and formaldehyde are as follows.

**Dioxane content**

| | |
|---|---|
| Excellent: | Less than 1 ppm |
| Pass: | 1 to 10 ppm |
| Poor: | More than 10 ppm |

**Formaldehyde content**

| | |
|---|---|
| Excellent: | Less than 1 ppm |
| Pass: | 1 to 10 ppm |
| Poor: | More than 10 ppm |

### Polymerization stability:

A polymer emulsion after polymerization was filtered with 80 mesh filter paper. Residue on the filter paper was washed with water, and dried. Its weight is shown by % based on the weight of the solid content of the emulsion.

### Mechanical stability:

50 g of a polymer emulsion was stirred in Marlon tester for 5 minutes at a number of revolution of 1,000 rpm under a load of 10 kg. Agglomerates formed were filtered with 80 mesh metal net, and the residue was washed with water, and dried. Its weight is shown by % based on the weight of the solid content of the emulsion.

### Particle diameter:

Particle diameter was measured with a dynamic light scattering particle size distribution measurement device (MICROTRAC UPA 9340, a product of Nikkiso Co., Ltd.), and was shown by µm.

### Foaming property:

An emulsion was diluted with water to a double volume, and 30cc of the diluted emulsion was introduced in a 100 ml Nessler tube. The tube was inverted 30 times. After allowing to stand the tube for 5 minutes, the amount of bubbles was measured, and was shown by ml.

### Luster of film:

A 0.5 mm (wet) emulsion coating was formed on a glass plate, and allowed to stand at room temperature for 24 hours to prepare a film. Luster of the film was visually evaluated by the three grades of "Excellent", "Pass" and "Poor".

**TABLE 2**

| | Emulsifier for emulsion polymerization | Dioxane content | Formaldehyde content | Polymerization stability (%) | Mechanical stability (%) | Particle diameter (µm) | Foaming property (ml) | Film luster |
|---|---|---|---|---|---|---|---|---|
| Example | Invention 1A | Excellent | Excellent | 0.21 | 0.41 | 0.161 | 0 | Excellent |
| | Invention 1B | Excellent | Excellent | 0.18 | 0.28 | 0.155 | 0 | Excellent |
| | Invention 1C | Excellent | Excellent | 0.16 | 0.15 | 0.158 | 1 | Excellent |
| | Invention 1D | Excellent | Excellent | 0.20 | 0.13 | 0.165 | 0 | Excellent |
| | Invention 1E | Excellent | Excellent | 0.32 | 0.03 | 0.179 | 0 | Excellent |
| | Invention 1F | Excellent | Excellent | 0.57 | 0.01 | 0.185 | 2 | Excellent |
| | Invention 1G | Excellent | Excellent | 0.08 | 0.30 | 0.154 | 1 | Excellent |
| | Invention 1H | Excellent | Excellent | 0.14 | 0.09 | 0.161 | 0 | Excellent |
| Comparative Example | Comparison 1A | Excellent | Poor | 0.21 | 0.23 | 0.158 | 1 | Excellent |
| | Comparison 1B | Excellent | Poor | 0.27 | 0.11 | 0.166 | 2 | Excellent |
| | Comparison 1C | Poor | Poor | 0.19 | 0.27 | 0.165 | 0 | Excellent |
| | Comparison 1D | Excellent | Poor | 0.29 | 0.13 | 0.177 | 1 | Pass |
| | Comparison 1E | Excellent | Poor | 0.37 | 2.81 | 0.171 | 31 | Poor |
| | Comparison 1F | Poor | Poor | 0.35 | 1.57 | 0.178 | 45 | Poor |

### USE EXAMPLE 2

135 parts of ion-exchanged water and 0.5 part of sodium hydrogencarbonate as a buffer were placed in a reactor equipped with a stirrer, a reflux condenser, a thermometer and a dropping funnel. Temperature was elevated to 80°C, and dissolved oxygen in water was removed with nitrogen gas. Separately, 75 parts of methyl methacrylate, 171 parts of ethyl acrylate, 4 parts of acrylic acid, 8 parts of an emulsifier for emulsion polymerization and 110 parts of ion-exchanged water were mixed to prepare a monomer emulsion. 40 parts of the monomer emulsion prepared above was added en bloc to the reactor, followed by stirring for 10 minutes. 0.5 part of ammonium persulfate as a polymerization initiator was added to the reactor, followed by stirring for 10 minutes. The remaining monomer emulsion was added dropwise to the reactor over 3 hours to conduct polymerization reaction. The reaction mixture was cooled to 40°C, and adjusted to pH 8 to 9 with aqueous ammonia to obtain a polymer emulsion.

The emulsifiers for emulsion polymerization used are shown in Table 3 below. 10% by weight in the emulsifier for emulsion polymerization was a linear alkylbenzenesulfonic acid sodium salt as an anionic emulsifier used in combination.

Polymerization stability, particle diameter, VOC amount, unreacted emulsifier amount and water resistance were evaluated on the polymer emulsion obtained. The evaluation methods of polymerization stability and particle diameter are the same as described above. Evaluation methods of VOC amount, unreacted emulsifier amount and water resistance are as follows. The results obtained are shown in Table 3 below. VOC amount:
VOC amount contained in a polymer emulsion was measured with head space GC. Evaluation criteria for the VOC amount are as follows.

| | |
|---|---|
| Excellent: | Less than 10 ppm |
| Pass: | 10 to 50 ppm |
| Poor: | More than 50 ppm |

### Unreacted emulsifier amount:

Methanol was added to a polymer emulsion to coagulate the polymer. After centrifugal separation treatment, its supernatant was used, and the unreacted emulsifier amount was measured with HPLC-MS method, and shown by %. Water resistance test:

0.5 mm thick polymer film was prepared on a glass plate.
The glass plate having the polymer film was dipped in water. Time until 4.5 point characters are not read through the polymer film was measured. Evaluation criteria for the water resistance are as follows.

| | |
|---|---|
| Excellent: | More than 300 hours |
| Pass: | 300 to 200 hours |
| Poor: | Less than 200 hours |

**TABLE 3**

| | Emulsifier for emulsion polymerization | Polymerization stability (%) | Particle diameter (µm) | VOC amount | Unreacted Emulsifier (%) | Water resistance |
|---|---|---|---|---|---|---|
| Example | Invention 1C | 0.17 | 0.158 | Excellent | 0 | Excellent |
| | Invention 1D | 0.25 | 0.167 | Excellent | 1 | Excellent |
| | Invention 1E | 0.40 | 0.173 | Excellent | 2 | Excellent |
| | Invention 1F | 0.61 | 0.180 | Excellent | 0 | Excellent |
| | Invention 1G | 0.20 | 0.163 | Excellent | 1 | Excellent |
| | Invention 1H | 0.28 | 0.170 | Excellent | 0 | Excellent |
| | Invention 1A/ Invention 1E (=1/1) | 0.23 | 0.163 | Excellent | 0 | Excellent |
| Comparative Example | Comparison 1A | 0.23 | 0.157 | Poor | 3 | Excellent |
| | Comparison 1C | 0.21 | 0.160 | Poor | 0 | Excellent |
| | Comparison 1D | 0.19 | 0.172 | Poor | 5 | Pass |
| | Comparison 1E | 0.38 | 0.171 | Poor | -* | Poor |
| | Comparison 1F | 0.30 | 0.177 | Poor | -* | Poor |

| | | | | | | |
|---|---|---|---|---|---|---|
| *: In the evaluation of unreacted emulsifier amount, "-" means that because of non-reactive, evaluation was not made. | | | | | | |

### USE EXAMPLE 3

250 parts of ion-exchanged water was placed in a reactor equipped with a stirrer, a reflux condenser, a thermometer and a dropping funnel. Temperature was elevated to 80°C, and dissolved oxygen in water was removed with nitrogen gas. 50 parts in a mixed monomer liquid prepared by dissolving 5 parts of an emulsifier for emulsion polymerization in 125 parts of butyl acrylate and 125 parts of 2-ethylhexyl acrylate was placed in the reactor. 0.5 part of ammonium persulfate was added to the reactor to conduct preceding polymerization. 205 parts of the remaining mixed monomer liquid was added dropwise to the reactor over 3 hours from 10 minutes after polymerization initiation to conduct polymerization. Continuously, the reaction mixture was aged at the polymerization temperature for 2 hours. The reaction mixture was cooled to 40°C, and adjusted to pH 8 to 9 with aqueous ammonia to obtain a polymer emulsion.

The emulsifiers for emulsion polymerization used are shown in Table 4 below. 10% by weight in the emulsifier for emulsion polymerization was a polyoxyethylene styrenated phenyletherphosphoric acid ester (EO 8 moles adduct) as an anionic emulsifier used in combination.

Polymerization stability, mechanical stability, unreacted emulsifier amount, heat coloring resistance and adhesion were evaluated on the polymer emulsion obtained. The evaluation methods of polymerization stability, mechanical stability and unreacted emulsifier amount are the same as described above. Evaluation methods of heat coloring resistance and adhesion are as follows. The results obtained are shown in Table 4 below.

### Heat coloring resistance:

A 0.5 mm thick polymer film was formed on a glass plate, and heat treated in a hot air dryer adjusted to 200°C for 30 minutes. Coloring of the polymer film was visually observed. Evaluation criteria for the heat coloring resistance are as follows.

| | |
|---|---|
| Excellent: | No coloring |
| Pass: | Coloring in pale yellow |
| Poor: | Coloring in dark brown |

### Adhesion:

An emulsion was applied to a PET film cut in a width of 5 cm in a thickness (dry) of 25 µm, and heat treated. The SUS plate was adhered to the emulsion coating, and pressed with a roll. The film was peeled such that the adhered area is 5 cm x 5 cm, and a 200 g weight was hung at the edge of the film. Time (second) until peeling the film was measured.

**TABLE 4**

| | Emulsifier for emulsion polymerization | Polymerization stability (%) | Mechanical stability (%) | Unreacted emulsifier (%) | Heat coloring resistance | Adhesion (second) |
|---|---|---|---|---|---|---|
| Example | Invention 1B | 0.21 | 0.37 | 0 | Excellent | 970 |
| | Invention 1C | 0.32 | 0.28 | 1 | Excellent | 910 |
| | Invention 1D | 0.39 | 0.11 | 1 | Excellent | 920 |
| | Invention 1E | 0.51 | 0.05 | 1 | Excellent | 890 |
| | Invention 1G | 0.30 | 0.25 | 0 | Excellent | 960 |
| | Invention 1H | 0.37 | 0.17 | 1 | Excellent | 940 |
| Comparative Example | Comparison 1A | 0.36 | 0.44 | 8 | Pass | 760 |
| | Comparison 1D | 0.50 | 0.98 | 9 | Pass | 710 |
| | Comparison 1E | 0.57 | 1.89 | -* | Poor | 620 |
| | Comparison 1F | 0.58 | 0.86 | -* | Poor | 570 |

| | | | | | | |
|---|---|---|---|---|---|---|
| *: In the evaluation of unreacted emulsifier amount, "-" means that because of non-reactive, evaluation was not made. | | | | | | |

### USE EXAMPLE 4

131 parts of ion-exchanged water and 0.5 part of sodium hydrogencarbonate as a buffer were placed in a reactor equipped with a stirrer, a reflux condenser, a thermometer and a dropping funnel. Temperature was elevated to 70°C, and dissolved oxygen in water was removed with nitrogen gas. Separately, 250 parts of vinyl acetate, 8 parts of an emulsifier for emulsion polymerization and 110 parts of ion-exchanged water were mixed to prepare a monomer emulsion. 40 parts of the monomer emulsion prepared above was added en bloc to the reactor, followed by stirring for 10 minutes. 0.5 part of ammonium persulfate as a polymerization initiator was added to the reactor, followed by stirring for 10 minutes. The remaining monomer emulsion was added dropwise to the reactor over 3 hours to conduct polymerization reaction. The reaction mixture was cooled to 40°C, and adjusted to pH 8 to 9 with aqueous ammonia to obtain a polymer emulsion. The emulsifiers for emulsion polymerization used are shown in Table 5 below.

Polymerization stability, particle diameter and adhesion were evaluated on the polymer emulsion obtained. The evaluation methods of polymerization stability and particle diameter are the same as described above. Evaluation method of adhesion is as follows. The results obtained are shown in Table 5 below.

### Adhesion:

An emulsion was applied to a plywood cut in a width of 5 cm in a thickness (dry) of 25 µm, and heat treated. A cotton cloth of a width of 5 cm was adhered to the emulsion coating, and pressed with a roll. The cloth was peeled such that the adhered area is 5 cm × 5 cm, and a 1 kg weight was hung at the edge of the cloth peeled. Time (second) until peeling the cloth was measured.

**TABLE 5**

| | Emulsifier for emulsion polymerization | Polymerization diameter stability | Particle diameter (µm) | Adhesion (second) |
|---|---|---|---|---|
| Example | Invention 1C | 0.86 | 0.178 | 660 |
| | Invention 1D | 0.61 | 0.188 | 690 |
| | Invention 1E | 0.39 | 0.164 | 760 |
| | Invention 1F | 0.17 | 0.156 | 790 |
| | Invention 1G | 0.79 | 0.189 | 660 |
| | Invention 1H | 0.65 | 0.171 | 710 |
| | Invention 1A/ Invention 1F (=1/1) | 0.43 | 0.166 | 720 |
| | Invention 1G/ Protective colloidal agent^{*1} | 0.40 | 0.160 | 780 |
| Comparative Example | Comparison 1A | 1.13 | 0.194 | 450 |
| | Comparison 1D | 0.88 | 0.181 | 500 |
| | Comparison 1E | 3.14 | 0.293 | -^{*2} |
| | Comparison 1F | 1.47 | 0.220 | 330 |

| | | | | |
|---|---|---|---|---|
| *1: Invention 1G/Protective colloidal agent=4/1 Protective colloidal agent: Partially saponified PVA, degree of saponification=90%, degree of polymerization of PVA=450 *2: In the adhesion evaluation, "-" means that because agglomerates generated next day after polymerization, test was not conducted. | | | | |

### Second Embodiment

### PRODUCTION EXAMPLE 9

200 parts of isotridecyl alcohol and sodium hydroxide as a catalyst were placed in a reactor equipped with a stirrer, a nitrogen introduction pipe and a thermometer, and dehydration was conducted at 105°C for 30 minutes under reduced pressure. After cooling to 90°C, 114 parts of allylglycidyl ether was added dropwise to the reactor, followed by aging at 90°C for 5 hours. The product obtained was heated to 120°C, and 370 parts of glycidol was added dropwise over 1 hour, followed by stirring for 2 hours. Water was added to the product, and the resulting mixture was passed through a cation exchange resin and an anion exchange resin to desalt, and then dehydrated under reduced pressure to obtain an emulsifier for emulsion polymerization 2A of the present invention.

### PRODUCTION EXAMPLE 10

200 parts of isotridecyl alcohol and a borotrifluoride ether complex as a catalyst were placed in a reactor equipped with a stirrer, a nitrogen introduction pipe and a thermometer, and dehydration was conducted at 105°C for 30 minutes under reduced pressure. After cooling to 90°C, 128 parts of methallylglycidyl ether was added dropwise to the reactor, followed by aging at 90°C for 5 hours. Potassium hydroxide as a catalyst was added to the product obtained. The resulting mixture was heated to 120°C, and 1,110 parts of glycidol was added dropwise over 1 hour, followed by stirring for 2 hours. Water was added to the product, and the resulting mixture was passed through a cation exchange resin and an anion exchange resin to desalt, and then dehydrated under reduced pressure to obtain an emulsifier for emulsion polymerization 2B of the present invention.

### PRODUCTION EXAMPLE 11

186 parts of lauryl alcohol and sodium hydroxide as a catalyst were placed in a reactor equipped with a stirrer, a nitrogen introduction pipe and a thermometer, and dehydration was conducted at 105°C for 30 minutes under reduced pressure. After cooling to 90°C, 114 parts of allylglycidyl ether was added dropwise to the reactor, followed by aging at 90°C for 5 hours. The product obtained was heated to 120°C, and 296 parts of glycidol was added dropwise over 1 hour, followed by stirring for 2 hours. Sulfuric acid was added to the product to neutralize, followed by dehydration. Precipitates were filtered off to obtain an emulsifier for emulsion polymerization 2C of the present invention.

### PRODUCTION EXAMPLE 12

An emulsifier for emulsion polymerization 2D of the present invention was obtained by conducting the reaction and purification under the same conditions as in Production Example 9, except for using 192 parts of Neodol 23 and 740 parts of glycidol.

### PRODUCTION EXAMPLE 13

87 parts of allyl alcohol and potassium hydroxide as a catalyst were placed in a reactor equipped with a stirrer, a nitrogen introduction pipe and a thermometer. 242 parts of laurylglycidyl ether was added dropwise to the reactor, and reaction was conducted at 80°C for 5 hours under stirring. Temperature was elevated to 120°C, and excess allyl alcohol was removed by reduced pressure. The product obtained was heated to 120°C, and 2,960 parts of glycidol was added dropwise over 1 hour, followed by stirring for 2 hours. Sulfuric acid was added to the product to neutralize, followed by dehydration. Precipitates were filtered off to obtain an emulsifier for emulsion polymerization 2E of the present invention.

### PRODUCTION EXAMPLE 14

An emulsifier for emulsion polymerization 2F of the present invention was obtained by conducting the reaction under the same conditions as in Production Example 10, except for using 172 parts of Exxal 11 and 5,920 parts of glycidol.

### PRODUCTION EXAMPLE 15

An emulsifier for emulsion polymerization 2G of the present invention was obtained by conducting the reaction and neutralization under the same conditions as in Production Example 11, except for using 158 parts of 2-propyl-1-heptanol and 222 parts of glycidol.

### PRODUCTION EXAMPLE 16

An emulsifier for emulsion polymerization 2H of the present invention was obtained by conducting the reaction under the same conditions as in Production Example 13, except for using 108 parts of methallyl alcohol, 214 parts of decylglycidyl ether and 740 parts of glycidol.

### PRODUCTION EXAMPLE 17

An emulsifier for emulsion polymerization 2I of the present invention was obtained by conducting the reaction and purification under the same conditions as in Production Example 9, except for using 305 parts of styrenated phenol (a mixture of mono-form, di-form and tri-form) and 370 parts of glycidol.

### EXAMPLES AND COMPARATIVE EXAMPLES

Emulsifiers for emulsion polymerization used in the Examples and Comparative Examples are shown in Table 6 below. Comparisons 2A to 2F are the conventional emulsifiers for emulsion polymerization, having added thereto the respective ethylene oxide having the chemical structure as shown in Table 6 below.

**TABLE 6**

| | | R^{*1} | R² | Number of glycerin unit |
|---|---|---|---|---|
| Invention | 2A | Isotridecyl group | Hydrogen atom | 5 |
| | 2B | Isotridecyl group | Methyl group | 15 |
| | 2C | Lauryl group | Hydrogen atom | 4 |
| | 2D | Neodol 23^{*2} residue | Hydrogen atom | 10 |
| | 2E | Lauryl group | Hydrogen atom | 40 |
| | 2F | Exxal 11^{*3} residue | Methyl group | 80 |
| | 2G | 2-Propyl-1-heptyl group | Hydrogen atom | 3 |
| | 2H | Decyl group | Methyl group | 10 |
| | 2I | Styrenated phenyl group | Hydrogen atom | 5 |
| Comparison | | Structure (EO represents ethylene oxide) | | |
| | 2A | 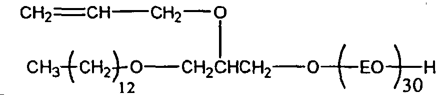 | | |
| | 2B | 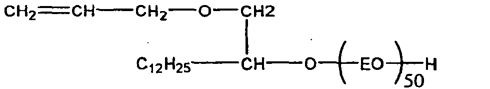 | | |
| | 2C | 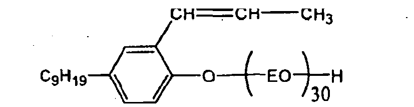 | | |
| | 2D | 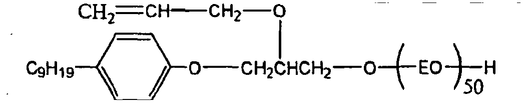 | | |
| | 2E | 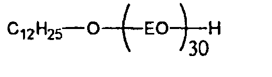 | | |
| | 2F | 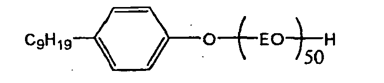 | | |

| | | | | |
|---|---|---|---|---|
| *1 : R¹=-CH₂-O-R *2: C₁₂-C₁₃ oxoalcohol, linearity about 80%, a product of Shell Chemicals. *3: C₁₀-C₁₂ oxoalcohol, highly branched type, a product of Exxon Chemical. | | | | |

### USE EXAMPLE 5

100 parts of butyl acrylate, 100 parts of styrene, 290 parts of ion-exchanged water and 10 parts of an emulsifier for emulsion polymerization were mixed to prepare a mixed monomer suspension, and dissolved oxygen was removed with nitrogen gas. 100 parts of the mixed monomer suspension was placed in a reactor equipped with a stirrer, a reflux condenser, a thermometer and a dropping funnel. Temperature was elevated to 80°C, 0.5 part of potassium persulfate was added to conduct preceding polymerization. 400 parts of the remaining mixed monomer suspension was added dropwise over 3 hours from 10 minutes after polymerization initiation. Continuously, the reaction mixture was aged at the polymerization temperature for 2 hours, and cooled to obtain a polymer emulsion.

The emulsifier for emulsion polymerization used is shown in Table 7 below. 10% by weight in the emulsifier for emulsion polymerization was laurylsulfuric acid ester sodium salt as an anionic emulsifier used in combination.

Content of dioxane and formaldehyde in the emulsifier for emulsion polymerization used; polymerization stability, mechanical stability, particle size and foaming property of the polymer emulsion obtained; and luster of the film were evaluated.
The evaluation methods are the same as in First Embodiment. The results obtained are shown in Table 7 below.

**TABLE 7**

| | Emulsifier for emulsion polymerization | Dioxane content | Formaldehyde content | Polymerization stability (%) | Mechanical stability (%) | Particle diameter (µm) | Foaming property (ml) | Film luster |
|---|---|---|---|---|---|---|---|---|
| Example | Invention 2A | Excellent | Excellent | 0.09 | 0.07 | 0.158 | 0 | Excellent |
| | Invention 2B | Excellent | Excellent | 0.06 | 0.03 | 0.167 | 1 | Excellent |
| | Invention 2C | Excellent | Excellent | 0.07 | 0.10 | 0.150 | 0 | Excellent |
| | Invention 2D | Excellent | Excellent | 0.10 | 0.05 | 0.160 | 0 | Excellent |
| | Invention 2E | Excellent | Excellent | 0.17 | 0.01 | 0.171 | 2 | Excellent |
| | Invention 2F | Excellent | Excellent | 0.29 | 0.01 | 0.188 | 1 | Excellent |
| | Invention 2G | Excellent | Excellent | 0.10 | 0.19 | 0.158 | 0 | Excellent |
| | Invention 2H | Excellent | Excellent | 0.15 | 0.07 | 0.163 | 1 | Excellent |
| | Invention 2I | Excellent | Excellent | 0.07 | 0.01 | 0.170 | 0 | Excellent |
| Comparative Example | Comparison 2A | Excellent | Poor | 0.20 | 0.19 | 0.158 | 1 | Excellent |
| | Comparison 2B | Excellent | Poor | 0.25 | 0.10 | 0.166 | 2 | Excellent |
| | Comparison 2C | Poor | Poor | 0.18 | 0.14 | 0.165 | 0 | Excellent |
| | Comparison 2D | Excellent | Poor | 0.16 | 0.08 | 0.177 | 1 | Pass |
| | Comparison 2E | Excellent | Poor | 0.35 | 2.89 | 0.171 | 29 | Poor |
| | Comparison 2F | Poor | Poor | 0.40 | 1.78 | 0.180 | 48 | Poor |

### USE EXAMPLE 6

135 parts of ion-exchanged water and 0.5 part of sodium hydrogencarbonate as a buffer were placed in a reactor equipped with a stirrer, a reflux condenser, a thermometer and a dropping funnel. Temperature was elevated to 80°C, and dissolved oxygen in water was removed with nitrogen gas. Separately, 75 parts of methyl methacrylate, 171 parts of ethyl acrylate, 4 parts of acrylic acid, 8 parts of an emulsifier for emulsion polymerization and 110 parts of ion-exchanged water were mixed to prepare a monomer emulsion. 40 parts of the monomer emulsion prepared above was added en bloc to the reactor, followed by stirring for 10 minutes. 0.5 part of ammonium persulfate as a polymerization initiator was added to the reactor, followed by stirring for 10 minutes. The remaining monomer emulsion was added dropwise to the reactor over 3 hours to conduct polymerization reaction. The reaction mixture was cooled to 40°C, and adjusted to pH 8 to 9 with aqueous ammonia to obtain a polymer emulsion.

The emulsifiers for emulsion polymerization used are shown in Table 8 below. 10% by weight in the emulsifier for emulsion polymerization was a linear alkylbenzenesulfonic acid sodium salt as an anionic emulsifier used in combination.

Polymerization stability, particle diameter, VOC amount, unreacted emulsifier amount and water resistance were evaluated on the polymer emulsion obtained. The evaluation methods are the same as in First Embodiment. The results obtained are shown in Table 8 below.

**TABLE 8**

| | Emulsifier for emulsion polymerization | Polymerization stability (%) | Particle diameter (µm) | VOC amount | Unreacted emulsifier (%) | Water resistance |
|---|---|---|---|---|---|---|
| Example | Invention 2A | 0.16 | 0.158 | Excellent | 0 | Excellent |
| | Invention 2C | 0.11 | 0.151 | Excellent | 0 | Excellent |
| | Invention 2D | 0.18 | 0.160 | Excellent | 1 | Excellent |
| | Invention 2E | 0.23 | 0.171 | Excellent | 2 | Excellent |
| | Invention 2G | 0.15 | 0.160 | Excellent | 0 | Excellent |
| | Invention 2H | 0.20 | 0.163 | Excellent | 1 | Excellent |
| | Invention 2I | 0.19 | 0.170 | Excellent | 0 | Excellent |
| Comparative Example | Comparison 2A | 0.23 | 0.158 | Poor | 3 | Excellent |
| | Comparison 2C | 0.21 | 0.165 | Poor | 0 | Excellent |
| | Comparison 2D | 0.19 | 0.177 | Poor | 5 | Pass |
| | Comparison 2E | 0.28 | 0.171 | Poor | -* | Poor |
| | Comparison 2F | 0.30 | 0.177 | Poor | -* | Poor |

| | | | | | | |
|---|---|---|---|---|---|---|
| *: In the evaluation of unreacted emulsifier amount, "-" means that because of non-reactive, evaluation was not made. | | | | | | |

### USE EXAMPLE 7

250 parts of ion-exchanged water was placed in a reactor equipped with a stirrer, a reflux condenser, a thermometer and a dropping funnel. Temperature was elevated to 80°C, and dissolved oxygen in water was removed with nitrogen gas. 50 parts in a mixed monomer liquid prepared by dissolving 5 parts of an emulsifier for emulsion polymerization in 125 parts of butyl acrylate and 125 parts of 2-ethylhexyl acrylate was placed in the reactor. 0.5 part of ammonium persulfate was added to the reactor to conduct preceding polymerization. 205 parts of the remaining mixed monomer liquid was added dropwise to the reactor over 3 hours from 10 minutes after polymerization initiation to conduct polymerization. Continuously, the reaction mixture was aged at the polymerization temperature for 2 hours. The reaction mixture was cooled to 40°C, and adjusted to pH 8 to 9 with aqueous ammonia to obtain a polymer emulsion.

The emulsifiers for emulsion polymerization used are shown in Table 9 below. 10% by weight in the emulsifier for emulsion polymerization was a polyoxyethylene styrenated phenyletherphosphoric acid ester (EO 8 moles adduct) as an anionic emulsifier used in combination.

Polymerization stability, mechanical stability, unreacted emulsifier amount, heat coloring resistance and adhesion were evaluated on the polymer emulsion obtained. The evaluation methods are the same as in First Embodiment. The results obtained are shown in Table 9 below.

**TABLE 9**

| | Emulsifier for emulsion polymerization | Polymerization stability (%) | Mechanical stability (%) | Unreacted emulsifier (%) | Heat coloring resistance | Adhesion (second) |
|---|---|---|---|---|---|---|
| Example | Invention 2A | 0.20 | 0.25 | 0 | Excellent | 990 |
| | Invention 2B | 0.32 | 0.08 | 1 | Excellent | 930 |
| | Invention 2C | 0.18 | 0.30 | 1 | Excellent | 960 |
| | Invention 2D | 0.37 | 0.05 | 1 | Excellent | 910 |
| | Invention 2G | 0.20 | 0.40 | 0 | Excellent | 950 |
| | Invention 2H | 0.41 | 0.15 | 1 | Excellent | 900 |
| | Invention 2I | 0.30 | 0.37 | 1 | Excellent | 1010 |
| Comparative Example | Comparison 2A | 0.36 | 0.44 | 7 | Pass | 740 |
| | Comparison 2D | 0.50 | 0.98 | 9 | Pass | 700 |
| | Comparison 2E | 0.57 | 1.89 | -* | Poor | 610 |
| | Comparison 2F | 0.58 | 0.86 | -* | Poor | 540 |

| | | | | | | |
|---|---|---|---|---|---|---|
| *: In the evaluation of unreacted emulsifier amount, "-" means that because of non-reactive, evaluation was not made. | | | | | | |

### USE EXAMPLE 8

131 parts of ion-exchanged water and 0.5 part of sodium hydrogencarbonate as a buffer were placed in a reactor equipped with a stirrer, a reflux condenser, a thermometer and a dropping funnel. Temperature was elevated to 70°C, and dissolved oxygen in water was removed with nitrogen gas. Separately, 250 parts of vinyl acetate, 8 parts of an emulsifier for emulsion polymerization and 110 parts of ion-exchanged water were mixed to prepare a monomer emulsion. 40 parts of the monomer emulsion prepared above was added en bloc to the reactor, followed by stirring for 10 minutes. 0.5 part of ammonium persulfate as a polymerization initiator was added to the reactor, followed by stirring for 10 minutes. The remaining monomer emulsion was added dropwise to the reactor over 3 hours to conduct polymerization reaction. The reaction mixture was cooled to 40°C, and adjusted to pH 8 to 9 with aqueous ammonia to obtain a polymer emulsion. The emulsifiers for emulsion polymerization used are shown in Table 10 below.

Polymerization stability, particle diameter and adhesion were evaluated on the polymer emulsion obtained. The evaluation methods of polymerization stability and particle diameter are the same as in First Embodiment. Evaluation method of adhesion is the same as in Use Example 4 of First Embodiment. The results obtained are shown in Table 10 below.

**TABLE 10**

| | Emulsifier for emulsion polymerization | Polymerization stability (%) | Particle diameter (µm) | Adhesion (second) |
|---|---|---|---|---|
| Example | Invention 2B | 0.66 | 0.177 | 690 |
| | Invention 2C | 0.85 | 0.189 | 650 |
| | Invention 2E | 0.39 | 0.166 | 750 |
| | Invention 2F | 0.12 | 0.157 | 770 |
| | Invention 2H | 0.59 | 0.172 | 700 |
| | Invention 2H/ Protective colloidal agent^{*1} | 0.41 | 0.163 | 740 |
| | Invention 2I | 0.73 | 0.179 | 730 |
| Comparative Example | Comparison 2A | 1.13 | 0.194 | 460 |
| | Comparison 2D | 0.88 | 0.181 | 500 |
| | Comparison 2E | 3.14 | 0.293 | -^{*2} |
| | Comparison 2F | 1.47 | 0.220 | 310 |

| | | | | |
|---|---|---|---|---|
| *1: Invention 2H/Protective colloidal agent=4/1 Protective colloidal agent: Partially saponified PVA, degree of saponification=90%, degree of polymerization of PVA=450 *2: In the adhesion evaluation, "-" means that because agglomerates generated next day after polymerization, test was not conducted. | | | | |

### Third Embodiment

### PRODUCTION EXAMPLE 18

87 parts of allyl alcohol and a borotrifluoride ether complex as a catalyst were placed in a reactor equipped with a stirrer, a nitrogen introduction pipe, a distillation apparatus and a thermometer, and 184 parts of α-olefin epoxide having 12 carbon atoms (Vikolox 12, a product of ARKEMA Inc.) was added dropwise thereto to conduct reaction at 80°C for 5 hours under stirring. Temperature was elevated to 120°C, and excess allyl alcohol was removed by reduced pressure to obtain an intermediate 3A. Potassium hydroxide as a catalyst was added to the intermediate 3A obtained, and temperature was elevated to 120°C. 148 parts of glycidol was added dropwise over 1 hour, followed by stirring for 2 hours, thereby obtaining an intermediate 3A' . To conduct purification, water was added to the intermediate 3A', and the resulting mixture was passed through a cation exchange resin and an anion exchange resin to desalt, followed by dehydration under reduced pressure. 100 parts of sulfamic acid was added to the intermediate 3A', and reaction was conducted at 120°C for 3 hours to perform sulfate esterification. Unreacted sulfamic acid was removed to obtain an emulsifier for emulsion polymerization 3A of the present invention.

### PRODUCTION EXAMPLE 19

108 parts of methallyl alcohol and a borotrifluoride ether complex as a catalyst were placed in a reactor equipped with a stirrer, a nitrogen introduction pipe, a distillation apparatus and a thermometer, and 196 parts of α-olefin epoxide having 12, 14 carbon atoms (AOE X24, a product of Daicel Chemical Industries, Ltd.) was added dropwise thereto to conduct reaction at 80°C for 5 hours under stirring. Temperature was elevated to 120°C, and excess methallyl alcohol was removed by reduced pressure to obtain an intermediate 3B. Potassium hydroxide as a catalyst was added to the intermediate 3B obtained, and temperature was elevated to 120°C. 370 parts of glycidol was added dropwise over 1 hour, followed by stirring for 2 hours, thereby obtaining an intermediate 3B'. Sulfuric acid was added to the intermediate 3B' to neutralize, followed by dehydration. Precipitates were filtered off. 100 parts of sulfamic acid was added to the intermediate 3B', and reaction was conducted at 120°C for 3 hours to perform sulfate esterification. Unreacted sulfamic acid was removed, followed by neutralization with monoethanolamine, thereby obtaining an emulsifier for emulsion polymerization 3B of the present invention.

### PRODUCTION EXAMPLE 20

Steps until obtaining an intermediate were conducted according the manner as in Production Example 18. 196 parts of α-olefin epoxide having 12, 14 carbon atoms (Epocizer M-24, a product of Dainippon Ink and Chemicals, Incorporated) was used in place of the α-olefin epoxide having 12 carbon atoms, thereby obtaining an intermediate 3C. 222 parts of glycidol was used to obtain an intermediate 3C'. 100 parts of sulfamic acid was added to the intermediate 3C', and reaction was conducted at 120°C for 3 hours to perform sulfate esterification. Unreacted sulfamic acid was removed, and the reaction mixture was dissolved in isopropyl alcohol. Sodium hydroxide in neutralization equivalent was added, followed by topping under reduced pressure, thereby obtaining an emulsifier for emulsion polymerization 3C of the present invention.

### PRODUCTION EXAMPLE 21

Steps until obtaining an intermediate were conducted according the manner as in Production Example 18. 212 parts of α-olefin epoxide having 14 carbon atoms (Vikolox 14, a product of ARKEMA Inc.) was used in place of the α-olefin epoxide having 12 carbon atoms, thereby obtaining an intermediate 3D. 740 parts of glycidol was used to obtain an intermediate 3D' . 45 parts of phosphoric anhydride was added to the intermediate 3D', and reaction was conducted at 80°C for 3 hours to perform phosphate esterification, thereby obtaining an emulsifier for emulsion polymerization 3D of the present invention.

### PRODUCTION EXAMPLE 22

An emulsifier for emulsion polymerization 3E of the present invention was obtained by conducting the reaction in the same manner as in Production Example 20, except for using 198 parts of branched α-olefin epoxide having 12 to 14 carbon atoms in place of α-olefin epoxide having 12, 14 carbon atoms, and using 296 parts of glycidol.

### PRODUCTION EXAMPLE 23

An emulsifier for emulsion polymerization 3F of the present invention was obtained by conducting the reaction in the same manner as in Production Example 18, except for using 108 parts of methallyl alcohol in place of allyl alcohol, and 240 parts of branched α-olefin epoxide having 16 carbon atoms in place of the α-olefin epoxide having 12 carbon atoms, and using 1,110 parts of glycidol.

### PRODUCTION EXAMPLE 24

Steps until obtaining an intermediate were conducted according the manner as in Production Example 18. 252 parts of α-olefin epoxide having 16, 18 carbon atoms (Epocizer M-68, a product of Dainippon Ink and Chemicals, Incorporated) was used in place of the α-olefin epoxide having 12 carbon atoms, thereby obtaining an intermediate 3G. 2,220 parts of glycidol was used to obtain an intermediate 3G'. 100 parts of maleic anhydride was added to the intermediate 3G', and reaction was conducted at 80°C for 2 hours, followed by sulfonation with anhydrous sodium sulfite, thereby obtaining an emulsifier for emulsion polymerization 3G of the present invention.

### PRODUCTION EXAMPLE 25

Steps until obtaining an intermediate were conducted according the manner as in Production Example 19. 252 parts of α-olefin epoxide having 16, 18 carbon atoms (AOE X68, a product of Daicel Chemical Industries, Ltd.) was used in place of the α-olefin epoxide having 12, 14 carbon atoms, thereby obtaining an intermediate 3H. 4, 440 parts of glycidol was used to obtain an intermediate 3H'. 116 parts of sodium monochloroacetate and sodium hydroxide as a catalyst were added to the intermediate 3H', and reaction was conducted at 80°C for 3 hours to perform ether carboxylation, followed by neutralization and purification, thereby obtaining an emulsifier for emulsion polymerization 3H of the present invention.

### EXAMPLES AND COMPARATIVE EXAMPLES

Emulsifiers for emulsion polymerization used in the Examples and Comparative Examples are shown in Table 11 below. Comparisons 3A to 3F are the conventional emulsifiers for emulsion polymerization, having added thereto the respective ethylene oxide having the chemical structure as shown in Table 11 below.

**TABLE 11**

| | | R^{*1} | R² | Number of glycerin unit | Anionic hydrophilic group |
|---|---|---|---|---|---|
| Invention | 3A | C10 alkyl group | Hydrogen atom | 2 | -SO₃NH₄ |
| | 3B | C10,12 alkyl group | Methyl group | 5 | -SO₃NH₄ (including monoethanol amine salt) |
| | 3C | C10,12 alkyl group | Hydrogen atom | 3 | -SO₃Na |
| | 3D | C12 alkyl group | Hydrogen atom | 10 | -PO₃H₂ and - -(PO₂H)_{½} |
| | 3E | Branched C10-12 alkyl group | Hydrogen atom | 4 | -SO₃Na |
| | 3F | Branched C14 alkyl group | Methyl group | 15 | -SO₃NH₄ |
| | 3G | C14, 16 alkyl group | Hydrogen atom | 30 | -COCH(SO₃Na)CH₂COONa |
| | 3H | C14, 16 alkyl group | Methyl group | 60 | -CH₂COONa |
| Comparison | | Structure (EO represents ethylene oxide) | | | |
| | 3A | 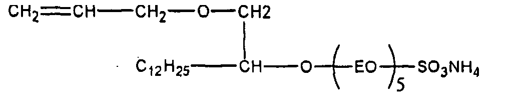 | | | |
| | 3B | 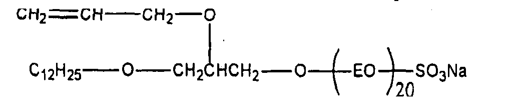 | | | |
| | 3C | 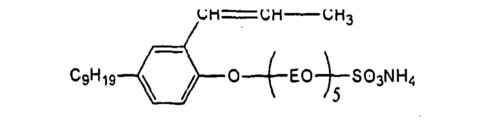 | | | |
| | 3D | 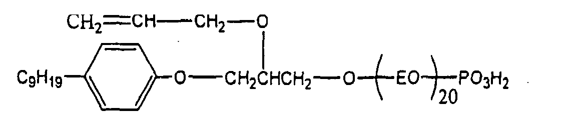 | | | |
| 3E | | 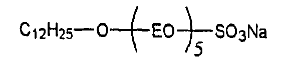 | | | |
| 3F | | 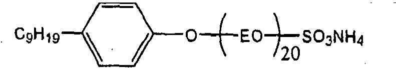 | | | |

| | | | | | |
|---|---|---|---|---|---|
| *1 : R¹=R | | | | | |

### USE EXAMPLE 9

100 parts of butyl acrylate, 100 parts of styrene, 290 parts of ion-exchanged water and 10 parts of an emulsifier for emulsion polymerization were mixed to prepare a mixed monomer suspension, and dissolved oxygen was removed with nitrogen gas. 100 parts of the mixed monomer suspension was placed in a reactor equipped with a stirrer, a reflux condenser, a thermometer and a dropping funnel. Temperature was elevated to 80°C, and 0.5 part of potassium persulfate was added to conduct preceding polymerization. 400 parts of the remaining mixed monomer suspension was added dropwise over 3 hours from 10 minutes after polymerization initiation to conduct polymerization. Continuously, the reaction mixture was aged at the polymerization temperature for 2 hours, and cooled to obtain a polymer emulsion. The emulsifier for emulsion polymerization used is shown in Table 12 below.

Content of dioxane and formaldehyde in the emulsifier for emulsion polymerization used; polymerization stability, mechanical stability, particle size and foaming property of the polymer emulsion obtained; and luster of the film were evaluated. The evaluation methods are the same as in First Embodiment. The results obtained are shown in Table 12 below.

**TABLE 12**

| | Emulsifier for emulsion polymerization | Dioxane content | Formaldehyde content | Polymerization stability (%) | Mechanical stability (%) | Particle diameter (µm) | Foaming property (ml) | Film luster |
|---|---|---|---|---|---|---|---|---|
| Example | Invention 3A | Excellent | Excellent | 0.03 | 0.17 | 0.142 | 1 | Excellent |
| | Invention 3B | Excellent | Excellent | 0.12 | 0.11 | 0.152 | 2 | Excellent |
| | Invention 3C | Excellent | Excellent | 0.06 | 0.16 | 0.145 | 0 | Excellent |
| | Invention 3D | Excellent | Excellent | 0.17 | 0.09 | 0.159 | 2 | Excellent |
| | Invention 3E | Excellent | Excellent | 0.08 | 0.12 | 0.147 | 0 | Excellent |
| | Invention 3F | Excellent | Excellent | 0.22 | 0.08 | 0.162 | 1 | Excellent |
| | Invention 3G | Excellent | Excellent | 0.30 | 0.03 | 0.169 | 0 | Excellent |
| | Invention 3H | Excellent | Excellent | 0.38 | 0.01 | 0.173 | 1 | Excellent |
| Comparative Example | Comparison 3A | Excellent | Poor | 0.15 | 0.33 | 0.150 | 2 | Excellent |
| | Comparison 3B | Excellent | Poor | 0.26 | 0.22 | 0.160 | 3 | Excellent |
| | Comparison 3C | Poor | Poor | 0.12 | 0.29 | 0.147 | 0 | Excellent |
| | Comparison 3D | Poor | Poor | 0.34 | 0.15 | 0.158 | 1 | Pass |
| | Comparison 3E | Excellent | Poor | 0.20 | 2.90 | 0.153 | 19 | Poor |
| | Comparison 3F | Poor | Poor | 0.42 | 2.40 | 0.168 | 40 | Poor |

### USE EXAMPLE 10

135 parts of ion-exchanged water and 0.5 part of sodium hydrogencarbonate as a buffer were placed in a reactor equipped with a stirrer, a reflux condenser, a thermometer and a dropping funnel. Temperature was elevated to 80°C, and dissolved oxygen in water was removed with nitrogen gas. Separately, 75 parts of methyl methacrylate, 171 parts of ethyl acrylate, 4 parts of acrylic acid, 8 parts of an emulsifier for emulsion polymerization and 110 parts of ion-exchanged water were mixed to prepare a monomer emulsion. 40 parts of the monomer emulsion prepared above was added en bloc to the reactor, followed by stirring for 10 minutes. 0.5 part of ammonium persulfate as a polymerization initiator was added to the reactor, followed by stirring for 10 minutes. The remaining monomer emulsion was added dropwise to the reactor over 3 hours to conduct polymerization reaction. The reaction mixture was cooled to 40°C, and adjusted to pH 8 to 9 with aqueous ammonia to obtain a polymer emulsion. The emulsifiers for emulsion polymerization used are shown in Table 13 below.

Polymerization stability, particle diameter, VOC amount, unreacted emulsifier amount and water resistance were evaluated on the polymer emulsion obtained. The evaluation methods are the same as in First Embodiment. The results obtained are shown in Table 13 below.

**TABLE 13**

| | Emulsifier for emulsion polymerization | Polymerization stability (%) | Particle diameter (µm) | VOC amount | Unreacted emulsifier (%) | Water resistance |
|---|---|---|---|---|---|---|
| Example | Invention 3A | 0.05 | 0.143 | Excellent | 1 | Excellent |
| | Invention 3B | 0.16 | 0.153 | Excellent | 2 | Excellent |
| | Invention 3C | 0.08 | 0.147 | Excellent | 0 | Excellent |
| | Invention 3D | 0.20 | 0.160 | Excellent | 4 | Excellent |
| | Invention 3E | 0.10 | 0.149 | Excellent | 3 | Excellent |
| | Invention 3F | 0.23 | 0.160 | Excellent | 0 | Excellent |
| | Invention 3G | 0.33 | 0.166 | Excellent | 1 | Excellent |
| | Invention 3H | 0.40 | 0.172 | Excellent | 0 | Excellent |
| Comparative Example | Comparison 3A | 0.22 | 0.152 | Poor | 5 | Excellent |
| | Comparison 3C | 0.21 | 0.150 | Poor | 5 | Excellent |
| | Comparison 3D | 0.41 | 0.177 | Poor | 7 | Pass |
| | Comparison 3E | 0.27 | 0.159 | Poor | -* | Poor |
| | Comparison 3F | 0.51 | 0.179 | Poor | -* | Poor |

| | | | | | | |
|---|---|---|---|---|---|---|
| *: In the evaluation of unreacted emulsifier amount, "-" means that because of non-reactive, evaluation was not made. | | | | | | |

### USE EXAMPLE 11

250 parts of ion-exchanged water was placed in a reactor equipped with a stirrer, a reflux condenser, a thermometer and a dropping funnel. Temperature was elevated to 80°C, and dissolved oxygen in water was removed with nitrogen gas. 50 parts in a mixed monomer liquid prepared by dissolving 5 parts of an emulsifier for emulsion polymerization in 125 parts of butyl acrylate and 125 parts of 2-ethylhexyl acrylate was placed in the reactor. 0.5 part of ammonium persulfate was added to the reactor to conduct preceding polymerization. 205 parts of the remaining mixed monomer liquid was added dropwise to the reactor over 3 hours from 10 minutes after polymerization initiation to conduct polymerization. Continuously, the reaction mixture was aged at the polymerization temperature for 2 hours. The reaction mixture was cooled to 40°C, and adjusted to pH 8 to 9 with aqueous ammonia to obtain a polymer emulsion. The emulsifiers for emulsion polymerization used are shown in Table 14 below.

Polymerization stability, mechanical stability, unreacted emulsifier amount, heat coloring resistance and adhesion were evaluated on the polymer emulsion obtained. The evaluation methods are the same as in First Embodiment. The results obtained are shown in Table 14 below.

**TABLE 14**

| | Emulsifier for emulsion polymerization | Polymerization stability (%) | Mechanical stability (%) | Unreacted emulsifier (%) | Heat coloring resistance | Adhesion (second) |
|---|---|---|---|---|---|---|
| Example | Invention 3A | 0.07 | 0.19 | 1 | Excellent | 990 |
| | Invention 3B | 0.10 | 0.13 | 1 | Excellent | 970 |
| | Invention 3C | 0.08 | 0.21 | 1 | Excellent | 1000 |
| | Invention 3D | 0.19 | 0.12 | 2 | Excellent | 960 |
| | Invention 3E | 0.06 | 0.17 | 0 | Excellent | 1010 |
| | Invention 3F | 0.16 | 0.10 | 2 | Excellent | 970 |
| | Invention 3G | 0.23 | 0.05 | 1 | Excellent | 930 |
| Comparative Example | Comparison 3A | 0.10 | 0.42 | 6 | Pass | 810 |
| | Comparison 3D | 0.30 | 0.22 | 5 | Pass | 770 |
| | Comparison 3E | 0.13 | 1.13 | -* | Poor | 620 |
| | Comparison 3F | 0.30 | 1.08 | -* | Poor | 570 |

| | | | | | | |
|---|---|---|---|---|---|---|
| *: In the evaluation of unreacted emulsifier amount, "-" means that because of non-reactive, evaluation was not made. | | | | | | |

### USE EXAMPLE 12

131 parts of ion-exchanged water and 0.5 part of sodium hydrogencarbonate as a buffer were placed in a reactor equipped with a stirrer, a reflux condenser, a thermometer and a dropping funnel. Temperature was elevated to 70°C, and dissolved oxygen in water was removed with nitrogen gas. Separately, 250 parts of vinyl acetate, 8 parts of an emulsifier for emulsion polymerization and 110 parts of ion-exchanged water were mixed to prepare a monomer emulsion. 40 parts of the monomer emulsion prepared above was added en bloc to the reactor, followed by stirring for 10 minutes. 0.5 part of ammonium persulfate as a polymerization initiator was added to the reactor, followed by stirring for 10 minutes. The remaining monomer emulsion was added dropwise to the reactor over 3 hours to conduct polymerization reaction. The reaction mixture was cooled to 40°C, and adjusted to pH 8 to 9 with aqueous ammonia to obtain a polymer emulsion. The emulsifiers for emulsion polymerization used are shown in Table 15 below.

Polymerization stability, particle diameter and adhesion were evaluated on the polymer emulsion obtained. The evaluation methods of polymerization stability and particle diameter are the same as in First Embodiment. Evaluation method of adhesion is the same as in Use Example 4 of First Embodiment. The results obtained are shown in Table 15 below.

**TABLE 15**

| | Emulsifier for emulsion polymerization | Polymerization stability (%) | Particle diameter (µm) | Adhesion (second) |
|---|---|---|---|---|
| Example | Invention 3A | 0.39 | 0.177 | 700 |
| | Invention 3B | 0.22 | 0.164 | 750 |
| | Invention 3D | 0.18 | 0.162 | 770 |
| | Invention 3E | 0.31 | 0.170 | 710 |
| | Invention 3F | 0.12 | 0.170 | 720 |
| | Invention 3G | 0.08 | 0.154 | 800 |
| | Invention 3H | 0.03 | 0.148 | 820 |
| | Invention 3C/ Protective colloidal agent^{*1} | 0.15 | 0.158 | 790 |
| Comparative Example | Comparison 3B | 0.73 | 0.175 | 530 |
| | Comparison 3C | 1.15 | 0.180 | 460 |
| | Comparison 3E | 2.00 | 0.296 | -^{*2} |
| | Comparison 3F | 0.75 | 0.182 | 340 |

| | | | | |
|---|---|---|---|---|
| *1: Invention 3C/Protective colloidal agent=4/1 Protective colloidal agent: Partially saponified PVA, degree of saponification=90%, degree of polymerization of PVA=450 *2: In the adhesion evaluation, "-" means that because agglomerates generated next day after polymerization, test was not conducted. | | | | |

### Fourth Embodiment

### PRODUCTION EXAMPLE 26

200 parts of isotridecyl alcohol and sodium hydroxide as a catalyst were placed in a reactor equipped with a stirrer, a nitrogen introduction pipe and a thermometer, and dehydration was conducted at 105°C for 30 minutes under reduced pressure. After cooling to 90°C, 114 parts of allylglycidyl ether was added dropwise to the reactor, followed by aging at 90°C for 5 hours. The product obtained was heated to 120°C, and 370 parts of glycidol was added dropwise over 1 hour, followed by stirring for 2 hours, thereby obtaining an intermediate 4A. Water was added to the intermediate 4A, and the resulting mixture was passed through a cation exchange resin and an anion exchange resin to purify, and then dehydrated under reduced pressure. 100 parts of sulfamic acid was added to the purified product of the intermediate 4A, and reaction was conducted at 120°C for 3 hours to perform sulfate esterification. Unreacted sulfamic acid was removed to obtain an emulsifier for emulsion polymerization 4A of the present invention.

### PRODUCTION EXAMPLE 27

200 parts of isotridecyl alcohol and a borotrifluoride ether complex as a catalyst were placed in a reactor equipped with a stirrer, a nitrogen introduction pipe and a thermometer, and dehydration was conducted at 105°C for 30 minutes under reduced pressure. After cooling to 90°C, 128 parts of methallylglycidyl ether was added dropwise to the reactor, followed by aging at 90°C for 5 hours. Potassium hydroxide as a catalyst was added to the product obtained. The resulting mixture was heated to 120°C, and 1,110 parts of glycidol was added dropwise over 1 hour, followed by stirring for 2 hours, thereby obtaining an intermediate 4B. Water was added to the intermediate 4B, and the resulting mixture was passed through a cation exchange resin and an anion exchange resin to purify, and then dehydrated under reduced pressure. 100 parts of sulfamic acid was added to the purified product of the intermediate 4B, and reaction was conducted at 120°C for 3 hours to perform sulfate esterification. Unreacted sulfamic acid was removed, and neutralized with monoethanolamine to obtain an emulsifier for emulsion polymerization 4B of the present invention.

### PRODUCTION EXAMPLE 28

186 parts of lauryl alcohol and sodium hydroxide as a catalyst were placed in a reactor equipped with a stirrer, a nitrogen introduction pipe and a thermometer, and dehydration was conducted at 105°C for 30 minutes under reduced pressure. After cooling to 90°C, 114 parts of allylglycidyl ether was added dropwise to the reactor, followed by aging at 90°C for 5 hours. The product obtained was heated to 120°C, and 370 parts of glycidol was added dropwise over 1 hour, followed by further stirring for 2 hours, thereby obtaining an intermediate 4C. Sulfuric acid was added to the intermediate 4C to neutralize, followed by dehydration. Precipitates were filtered off. 100 parts of sulfamic acid was added to the filtrate, of the intermediate 4C and reaction was conducted at 120°C for 3 hours to perform sulfate esterification. Unreacted sulfamic acid was removed, and the reaction mixture was dissolved in isopropyl alcohol. Sodium hydroxide in neutralization equivalent was added, followed by topping under reduced pressure, thereby obtaining an emulsifier for emulsion polymerization 4C of the present invention.

### PRODUCTION EXAMPLE 29

192 parts of Neodol 23 and sodium hydroxide as a catalyst were placed in a reactor equipped with a stirrer, a nitrogen introduction pipe and a thermometer, and dehydration was conducted at 105°C for 30 minutes under reduced pressure. After cooling to 90°C, 114 parts of allylglycidyl ether was added dropwise to the reactor, followed by aging at 90°C for 5 hours. The product obtained was heated to 120°C, and 1,110 parts of glycidol was added dropwise over 1 hour, followed by stirring for 2 hours, thereby obtaining an intermediate 4D. Water was added to the intermediate 4D, and the resulting mixture was passed through a cation exchange resin and an anion exchange resin to purify, and then dehydrated under reduced pressure. 45 parts of diphosphorus pentoxide was added to the purified product of the intermediate 4D, and reaction was conducted at 80°C for 3 hours to perform phosphate esterification, thereby obtaining an emulsifier for emulsion polymerization 4D of the present invention.

### PRODUCTION EXAMPLE 30

87 parts of allyl alcohol and potassium hydroxide as a catalyst were placed in a reactor equipped with a stirrer, a nitrogen introduction pipe and a thermometer. 242 parts of laurylglycidyl ether was added dropwise to the reactor, followed by conducting reaction at 80°C for 5 hours under stirring. Temperature was elevated to 120°C, and excess allyl alcohol was removed by reduced pressure. The product obtained was heated to 120°C, and 2,220 parts of glycidol was added dropwise over 1 hour, followed by stirring for 2 hours, thereby obtaining an intermediate 4E. Sulfuric acid was added to the intermediate 4E to neutralize, followed by dehydration. The precipitates were filtered off. 100 parts of maleic anhydride was added to the filtrate of the intermediate 4E, and reaction was conducted at 80°C for 2 hours, followed by sulfonation with anhydrous sodium sulfite, thereby obtaining an emulsifier for emulsion polymerization 4E of the present invention.

### PRODUCTION EXAMPLE 31

172 parts of Exxal 11 and a borotrifluoride ether complex as a catalyst were placed in a reactor equipped with a stirrer, a nitrogen introduction pipe and a thermometer, and dehydration was conducted at 105°C for 30 minutes under reduced pressure. After cooling to 90°C, 128 parts of methallylglycidyl ether was added dropwise to the reactor, followed by aging at 90°C for 5 hours. Potassium hydroxide as a catalyst was added to the product obtained. The resulting mixture was heated to 120°C, and 4,440 parts of glycidol was added dropwise over 1 hour, followed by stirring for 2 hours, thereby obtaining an intermediate 4F. Water was added to the intermediate 4F, and the resulting mixture was passed through a cation exchange resin and an anion exchange resin to purify, and then dehydrated under reduced pressure. 116 parts of sodium monochloroacetate and sodium hydroxide as a catalyst were added to the purified product of the intermediate 4F, and reaction was conducted at 80°C for 3 hours to perform ether carboxylation. The reaction mixture was purified and neutralized to obtain an emulsifier for emulsion polymerization 4F of the present invention.

### PRODUCTION EXAMPLE 32

An emulsifier for emulsion polymerization 4G of the present invention was obtained by conducting the reaction under the same conditions as in Production Example 28, except for using 158 parts of 2-propyl-1-heptanol and 222 parts of glycidol.

### PRODUCTION EXAMPLE 33

108 parts of methallyl alcohol and potassium hydroxide as a catalyst were placed in a reactor equipped with a stirrer, a nitrogen introduction pipe and a thermometer. 214 parts of decylglycidyl ether was added dropwise to the reactor, and reaction was conducted at 80°C for 5 hours under stirring. Temperature was elevated to 120°C, and excess methallyl alcohol was removed by reduced pressure. The product obtained was heated to 120°C, and 740 parts of glycidol was added dropwise over 1 hour, followed by stirring for 2 hours, thereby obtaining an intermediate 4H. Sulfuric acid was added to the intermediate 4H to neutralize, followed by dehydration. Precipitates were filtered off. 100 parts of sulfamic acid was added to the filtrate of the intermediate 4H, and reaction was conducted at 120°C for 3 hours, thereby performing sulfate esterification. Unreacted sulfamic acid was removed to obtain an emulsifier for emulsion polymerization 4H of the present invention.

### PRODUCTION EXAMPLE 34

An emulsifier for emulsion polymerization 4I of the present invention was obtained by conducting the reaction under the same conditions as in Production Example 29, except for using 305 parts of styrenated phenol (mixture of mono-form, di-form and tri-form) and 1,110 parts of glycidol.

### EXAMPLES AND COMPARATIVE EXAMPLES

Emulsifiers for emulsion polymerization used in the Examples and Comparative Examples are shown in Table 16 below. Comparisons 4A to 4F are the conventional emulsifiers for emulsion polymerization, having added thereto the respective ethylene oxide having the chemical structure as shown in Table 16 below.

**TABLE 16**

| | | R*¹ | R² | Number of glycerin unit | Anionic hydrophilic group |
|---|---|---|---|---|---|
| Invention | 4A | Isotridecyl group | Hydrogen atom | 5 | -SO₃NH₄ |
| | 4B | Isotridecyl group | Methyl group | 15 | - SO₃NH₄ (including monoethanol amine salt) |
| | 4C | Lauryl group | Hydrogen atom | 5 | -SO₃Na |
| | 4D 4E | Neodol 23*² residue | Hydrogen atom | 15 | -PO₃H₂ and - (PO₂H)_{½} |
| | | Lauryl group | Hydrogen atom | 30 | -COCH (SO₃Na) CH₂COONa |
| | 4F | Exxal 11*³ residue | Methyl group | 60 | -CH₂COONa |
| | 4G | 2-Propyl-1-hptyl group | Hydrogen atom | 3 | -SO₃Na |
| | 4H | Decyl group | Methyl group | 10 | -SO₃NH₄ |
| | 4I | Styrenated phenyl group | Hvdroaen atom | 15 | -PO₂H₂ and - (PO₂H)_{1/2} |
| Comparison | | Structure (EO represents ethylene oxide) | | | |
| | 4A | 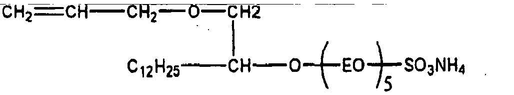 | | | |
| | 4B | 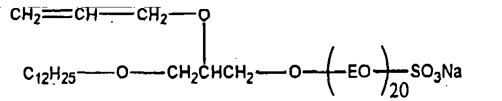 | | | |
| | 4C | 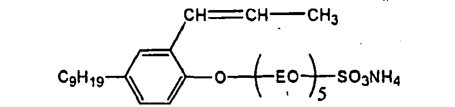 | | | |
| | 4D | 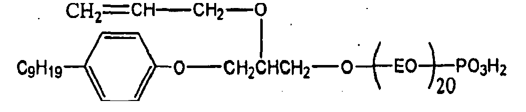 | | | |
| | 4E | 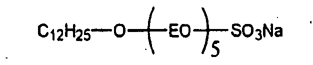 | | | |
| | 4F | 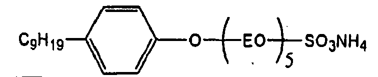 | | | |

| | | | | | |
|---|---|---|---|---|---|
| * 1 : R¹=-CH₂-O-R *2: C₁₂-C₁₃ oxoalcohol, linearity about 80%, a product of Shell Chemicals. *3: C₁₀-C₁₂ oxoalcohol, highly branched type, a product of Exxon Chemical. | | | | | |

### USE EXAMPLE 13

100 parts of butyl acrylate, 100 parts of styrene, 290 parts of ion-exchanged water and 10 parts of an emulsifier for emulsion polymerization were mixed to prepare a mixed monomer suspension, and dissolved oxygen was removed with nitrogen gas. 100 parts of the mixed monomer suspension was placed in a reactor equipped with a stirrer, a reflux condenser, a thermometer and a dropping funnel. Temperature was elevated to 80°C, 0.5 part of potassium persulfate was added to conduct preceding polymerization. 400 parts of the remaining mixed monomer suspension was added dropwise over 3 hours from 10 minutes after polymerization initiation. Continuously, the reaction mixture was aged at the polymerization temperature for 2 hours, and cooled to obtain a polymer emulsion. The emulsifier for emulsion polymerization used is shown in Table 17 below.

Content of dioxane and formaldehyde in the emulsifier for emulsion polymerization used; polymerization stability, mechanical stability, particle size and foaming property of the polymer emulsion obtained; and luster of the film were evaluated.
The evaluation methods are the same as in First Embodiment. The results obtained are shown in Table 17 below.

**TABLE 17**

| | Emulsifier for emulsion polymerization | Dioxane content | Formaldehyde content | Polymerization stability (%) | Mechanical stability (%) | Particle diameter (µm) | Foaming property (ml) | Film luster |
|---|---|---|---|---|---|---|---|---|
| Example | Invention 4A | Excellent | Excellent | 0.06 | 0.20 | 0.143 | 0 | Excellent |
| | Invention 4B | Excellent | Excellent | 0.14 | 0.12 | 0.152 | 1 | Excellent |
| | Invention 4C | Excellent | Excellent | 0.06 | 0.25 | 0.141 | 0 | Excellent |
| | Invention 4D | Excellent | Excellent | 0.25 | 0.13 | 0.155 | 0 | Excellent |
| | Invention 4E | Excellent | Excellent | 0.22 | 0.05 | 0.162 | 2 | Excellent |
| | Invention 4F | Excellent | Excellent | 0.49 | 0.03 | 0.186 | 1 | Excellent |
| | Invention 4G | Excellent | Excellent | 0.07 | 0.27 | 0.148 | 0 | Excellent |
| | Invention 4H | Excellent | Excellent | 0.14 | 0.15 | 0.160 | 1 | Excellent |
| | Invention 41 | Excellent | Excellent | 0.19 | 0.04 | 0.172 | 0 | Excellent |
| Comparative Example | Comparison 4A | Excellent | Poor | 0.16 | 0.34 | 0.147 | 1 | Excellent |
| | Comparison 4B | Excellent | Poor | 0.25 | 0.21 | 0.159 | 2 | Excellent |
| | Comparison 4C | Poor | Poor | 0.13 | 0.28 | 0.145 | 0 | Excellent |
| | Comparison 4D | Excellent | Poor | 0.33 | 0.16 | 0.156 | 1 | Pass |
| | Comparison 4E | Excellent | Poor | 0.19 | 2.93 | 0.152 | 17 | Poor |
| | Comparison 4F | Poor | Poor | 0.40 | 2.38 | 0.168 | 38 | Poor |

### USE EXAMPLE 14

135 parts of ion-exchanged water and 0.5 part of sodium hydrogencarbonate as a buffer were placed in a reactor equipped with a stirrer, a reflux condenser, a thermometer and a dropping funnel. Temperature was elevated to 80°C, and dissolved oxygen in water was removed with nitrogen gas. Separately, 75 parts of methyl methacrylate, 171 parts of ethyl acrylate, 4 parts of acrylic acid, 8 parts of an emulsifier for emulsion polymerization and 110 parts of ion-exchanged water were mixed to prepare a monomer emulsion. 40 parts of the monomer emulsion prepared above was added en bloc to the reactor, followed by stirring for 10 minutes. 0.5 part of ammonium persulfate as a polymerization initiator was added to the reactor, followed by stirring for 10 minutes. The remaining monomer emulsion was added dropwise to the reactor over 3 hours to conduct polymerization reaction. The reaction mixture was cooled to 40°C, and adjusted to pH 8 to 9 with aqueous ammonia to obtain a polymer emulsion. The emulsifiers for emulsion polymerization used are shown in Table 18 below.

Polymerization stability, particle diameter, VOC amount, unreacted emulsifier amount and water resistance were evaluated on the polymer emulsion obtained. The evaluation methods are the same as in First Embodiment. The results obtained are shown in Table 18 below.

**TABLE 18**

| | Emulsifier for emulsion polymerization | Polymerization stability (%) | Particle diameter (µm) | VOC amount | Unreacted emulsifier (%) | Water resistance |
|---|---|---|---|---|---|---|
| Example | Invention 4A | 0.13 | 0.144 | Excellent | 0 | Excellent |
| | Invention 4C | 0.15 | 0.148 | Excellent | 0 | Excellent |
| | Invention 4D | 0.25 | 0.162 | Excellent | 0 | Excellent |
| | Invention 4E | 0.38 | 0.177 | Excellent | 1 | Excellent |
| | Invention 4G | 0.14 | 0.151 | Excellent | 0 | Excellent |
| | Invention 4H | 0.26 | 0.166 | Excellent | 1 | Excellent |
| | Invention 4I | 0.32 | 0.170 | Excellent | 1 | Excellent |
| Comparative Example | Comparison 4A | 0.23 | 0.153 | Poor | 5 | Excellent |
| | Comparison 4C | 0.21 | 0.150 | Poor | 2 | Excellent |
| | Comparison 4D | 0.40 | 0.176 | Poor | 8 | Pass |
| | Comparison 4E | 0.27 | 0.159 | Poor | -* | Poor |
| | Comparison 4F | 0.50 | 0.179 | Poor | -* | Poor |

| | | | | | | |
|---|---|---|---|---|---|---|
| *: In the evaluation of unreacted emulsifier amount, "-" means that because of non-reactive, evaluation was not made. | | | | | | |

### USE EXAMPLE 15

250 parts of ion-exchanged water was placed in a reactor equipped with a stirrer, a reflux condenser, a thermometer and a dropping funnel. Temperature was elevated to 80°C, and dissolved oxygen in water was removed with nitrogen gas. 50 parts in a mixed monomer liquid prepared by dissolving 5 parts of an emulsifier for emulsion polymerization in 125 parts of butyl acrylate and 125 parts of 2-ethylhexyl acrylate was placed in the reactor. 0.5 part of ammonium persulfate was added to the reactor to conduct preceding polymerization. 205 parts of the remaining mixed monomer liquid was added dropwise to the reactor over 3 hours after 10 minutes from polymerization initiation to conduct polymerization. Continuously, the reaction mixture was aged at the polymerization temperature for 2 hours. The reaction mixture was cooled to 40°C, and adjusted to pH 8 to 9 with aqueous ammonia to obtain a polymer emulsion.
The emulsifiers for emulsion polymerization used are shown in Table 19 below.

In Inventions 4A to 4D and Comparisons 4A, 4D, 4E and 4F, a polyoxyethylene lauryl ether (EO 30 moles adduct) was used as a nonionic emulsifier to be used in combination in an amount of 10% by weight in the emulsifier for emulsion polymerization, and In Inventions 4G to 4I, laurylsulfuric acid ester sodium salt was used as a anionic emulsifier to be used in combination in an amount of 10% by weight in the emulsifier for emulsion polymerization.

Polymerization stability, mechanical stability, unreacted emulsifier amount, heat coloring resistance and adhesion were evaluated on the polymer emulsion obtained. The evaluation methods are the same as in First Embodiment. The results obtained are shown in Table 19 below.

**TABLE 19**

| | Emulsifier for emulsion polymerization | Polymerization stability (%) | Mechanical stability (%) | Unreacted emulsifier (%) | Heat coloring resistance | Adhesion (second) |
|---|---|---|---|---|---|---|
| Example | Invention 4A | 0.06 | 0.23 | 0 | Excellent | 1050 |
| | Invention 4B | 0.14 | 0.05 | 1 | Excellent | 970 |
| | Invention 4C | 0.08 | 0.27 | 1 | Excellent | 1000 |
| | Invention 4D | 0.17 | 0.11 | 0 | Excellent | 940 |
| | Invention 4G | 0.03 | 0.33 | 0 | Excellent | 950 |
| | Invention 4H | 0.15 | 0.14 | 0 | Excellent | 910 |
| | Invention 41 | 0.07 | 0.12 | 1 | Excellent | 1020 |
| Comparative Example | Comparison 4A | 0.10 | 0.42 | 6 | Pass | 800 |
| | Comparison 4D | 0.30 | 0.22 | 5 | Pass | 750 |
| | Comparison 4E | 0.13 | 1.13 | -* | Poor | 630 |
| | Comparison 4F | 0.30 | 1.08 | -* | Poor | 580 |

| | | | | | | |
|---|---|---|---|---|---|---|
| *: In the evaluation of unreacted emulsifier amount, "-" means that because of non-reactive, evaluation was not made. | | | | | | |

### USE EXAMPLE 16

131 parts of ion-exchanged water and 0.5 part of sodium hydrogencarbonate as a buffer were placed in a reactor equipped with a stirrer, a reflux condenser, a thermometer and a dropping funnel. Temperature was elevated to 70°C, and dissolved oxygen in water was removed with nitrogen gas. Separately, 250 parts of vinyl acetate, 8 parts of an emulsifier for emulsion polymerization and 110 parts of ion-exchanged water were mixed to prepare a monomer emulsion. 40 parts of the monomer emulsion prepared above was added en bloc to the reactor, followed by stirring for 10 minutes. 0.5 part of ammonium persulfate as a polymerization initiator was added to the reactor, followed by stirring for 10 minutes. The remaining monomer emulsion was added dropwise to the reactor over 3 hours to conduct polymerization reaction. The reaction mixture was cooled to 40°C, and adjusted to pH 8 to 9 with aqueous ammonia to obtain a polymer emulsion. The emulsifiers for emulsion polymerization used are shown in Table 20 below.

Polymerization stability, particle diameter and adhesion were evaluated on the polymer emulsion obtained. The evaluation methods of polymerization stability and particle diameter are the same as in First Embodiment. Evaluation method of adhesion is the same as in Use Example 4 in First Embodiment. The results obtained are shown in Table 20 below.

**TABLE 20**

| | Emulsifier for emulsion polymerization | Polymerization stability | Particle diameter (µm) | Adhesion (second) |
|---|---|---|---|---|
| Example | Invention 4B | 0.36 | 0.162 | 710 |
| | Invention 4C | 0.55 | 0.177 | 740 |
| | Invention 4E | 0.20 | 0.157 | 760 |
| | Invention 4F | 0.08 | 0.151 | 800 |
| | Invention 4H | 0.34 | 0.172 | 740 |
| | Invention 4I | 0.40 | 0.160 | 780 |
| | Invention 4A/ Protective colloidal agent^{*1} | 0.18 | 0.154 | 770 |
| Comparative Example | Comparison 4A | 1.20 | 0.184 | 470 |
| | Comparison 4D | 0.90 | 0.177 | 530 |
| | Comparison 4E | 1.99 | 0.291 | -^{*2} |
| | Comparison 4F | 0.72 | 0.185 | 330 |

| | | | | |
|---|---|---|---|---|
| *1: Invention 4A/Protective colloidal agent=4/1 Protective colloidal agent: Partially saponified PVA, degree of saponification=90%, degree of polymerization of PVA=450 *2: In the adhesion evaluation, "-" means that because agglomerates generated next day after polymerization, test was not conducted. | | | | |

From the above test results, the emulsifier for emulsion polymerization according to the present invention is that the amount of aldehyde or dioxane contained in the emulsifier is extremely small, polymerization stability and mechanical stability of the emulsion are good, the amount of the unreacted emulsifier is extremely small, and generation of harmful by-products such as VOC can greatly be reduced. Further, since the amount of the unreacted emulsifier is small, it is apparent that various properties, such as film luster, adhesion and water resistance, of the polymer emulsion obtained are superior to those using the conventional emulsifier for emulsion polymerization.

The emulsifier for emulsion polymerization of the present invention has excellent polymerizability and copolymerizability of the monomer at the time of emulsion polymerization, is liable to be incorporated into a polymer composition, and exhibits extremely excellent effect in improvement of various properties such as water resistance, adhesion, heat resistance and weather resistance of the film obtained from the polymer emulsion obtained by polymerizing using the emulsifier for emulsion polymerization. In addition, foaming property, mechanical stability and the like of the polymer emulsion are remarkably improved. Further, the polymer emulsion having greatly reduced harmful substances such as dioxane and aldehydes can be obtained.

### INDUSTRAL APPLICABILITY

The polymer emulsion obtained by the present invention is suitable to woods, metals, papers, fabrics, concretes and the like as an adhesive, a covering material, or an impregnating reinforcement. Further, the polymer taken out of the emulsion or latex can be used to, for example, a modifier of resins, rubbers and polymers.

## Claims

1. An emulsifier for emulsion polymerization, comprising a compound represented by the following general formula (1): 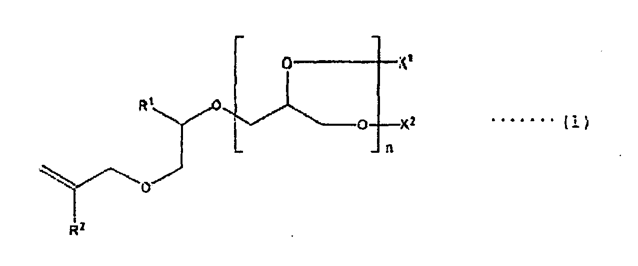 wherein R¹ represents a substituent represented by R or -CH₂-O-R wherein R is selected from the group consisting of decyl, secondary decyl, undecyl, secondary undecyl, dodecyl, secondary dodecyl, tridecyl, isotridecyl, secondary tridecyl, tetradecyl, secondary tetradecyl, hexadecyl, secondary hexadecyl, 2-butyloctyl, 2-butyldecyl, 2-hexyloctyl, 2-hexyldecyl and styrenated phenyl, and additionally -O-R may be a residue of an alcohol selected from the group consisting of lauryl alcohol, myristyl alcohol, cetyl alcohol, isodecanol, isoundecanol, isododecanol, isotridecanol, 2-propyl-1-octanol, 2-propyl-1-heptanol, 4-methyl-2-propyl-1-hexanol and 2-propyl-5-methyl-1-hexanol; R² represents a hydrogen atom or a methyl group; n represents from 1 to 200; X¹ and X² each represents a hydrogen atom, a hydrocarbon group or an anionic hydrophilic group.

2. The emulsifier for emulsion polymerization as claimed in claim 1, wherein the general formula (1) has at least one of anionic hydrophilic groups represented by the following general formulae (2) to (6) as the anionic hydrophilic group: 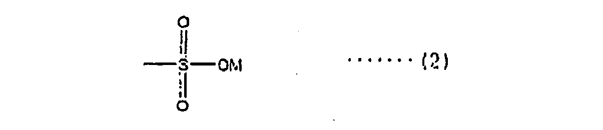 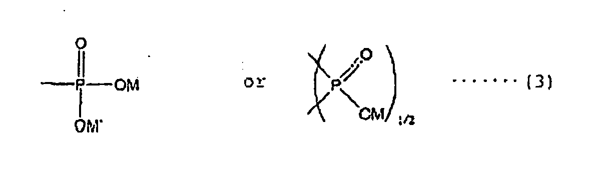 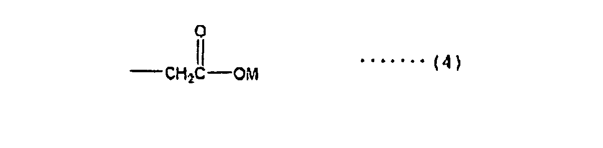 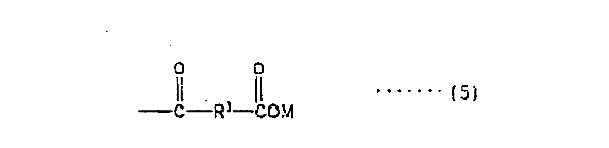 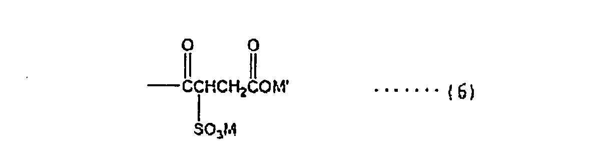 wherein R³ represents a residue that a carboxyl group is eliminated from a dibasic acid; M and M' which may be the same or different each represents a hydrogen atom, a metallic atom, ammonium or a hydrocarbon group.

3. An emulsifier for emulsion polymerization, comprising a mixture of the compound as claimed in claim 1 or 2 with at least one surfactant selected from the group consisting of nonionic surfactants, anionic surfactants, and cationic surfactants, other than the compound.

4. A production method of a polymer emulsion, which comprises polymerizing monomers using the emulsifier for emulsion polymerization as claimed in any one of claims 1 to 3 in an amount of from 0.1 to 20% by weight based on the weight of the entire monomers in an aqueous medium, or adding the emulsifier for emulsion polymerization to a polymer after polymerization of the monomers.

5. A polymer emulsion obtained by the production method of a polymer emulsion as claimed in claim 4.

## Patentansprüche

1. Emulgiermittel für die Emulsionspolymerisation, welches eine Verbindung der folgenden allgemeinen Formel (1) umfasst: 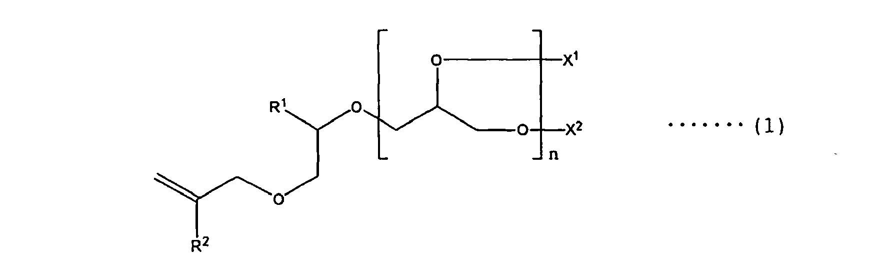 worin R¹ einen durch R oder -CH₂-O-R dargestellten Substituenten darstellt, wobei R aus der Gruppe ausgewählt ist, die aus Decyl, sekundärem Decyl, Undecyl, sekundärem Undecyl, Dodecyl, sekundärem Dodecyl, Tridecyl, Isotridecyl, sekundärem Tridecyl, Tetradecyl, sekundärem Tetradecyl, Hexadecyl, sekundärem Hexadecyl, 2-Butyloctyl, 2-Butyldecyl, 2-Hexyloctyl, 2-Hexyldecyl und styrolisiertem Phenyl besteht, und außerdem -O-R ein Rest eines Alkohols sein kann, der aus der Gruppe ausgewählt ist, die aus Laurylalkohol, Myristylalkohol, Cetylalkohol, Isodecanol, Isoundecanol, Isododecanol, Isotridecanol, 2-Propyl-1-Octanol, 2-Propyl-1-heptanol, 4-Methyl-2-propyl-1-hexanol und 2-Propyl-5-methyl-1-hexanol besteht; R² ein Wasserstoffatom oder eine Methylgruppe darstellt; n 1 bis 200 darstellt; X¹ und X² jeweils ein Wasserstoffatom, eine Kohlenwasserstoffgruppe oder eine anionische hydrophile Gruppe darstellen.

2. Emulgiermittel für die Emulsionspolymerisation nach Anspruch 1, wobei die allgemeine Formel (1) mindestens eine der durch die folgenden allgemeinen Formeln (2) bis (6) dargestellten anionischen hydrophilen Gruppen als die anionische hydrophile Gruppe aufweist: 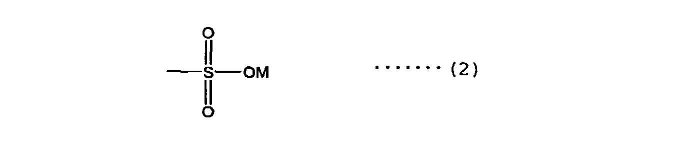 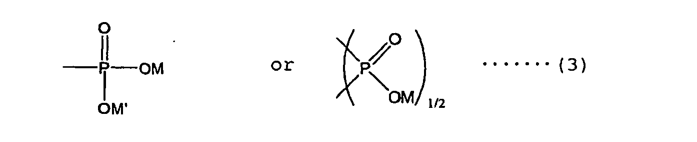 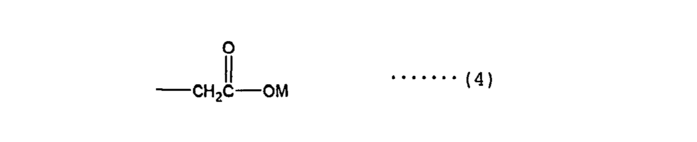 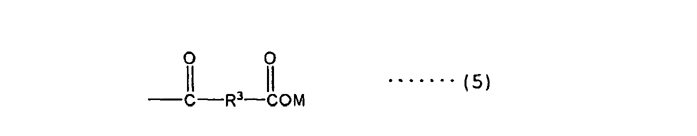 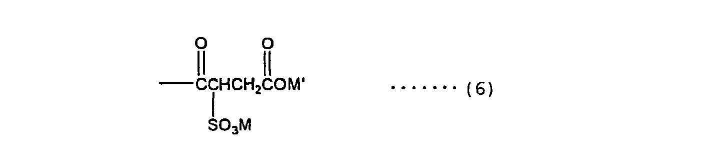 worin R³ einen Rest einer zweibasigen Säure darstellt, von der eine Carboxygruppe eliminiert worden ist; M und M', die gleich oder verschieden sein können, jeweils ein Wasserstoffatom, ein metallisches Atom, Ammonium oder eine Kohlenwasserstoffgruppe darstellen.

3. Emulgiermittel für die Emulsionspolymerisation, welches ein Gemisch der Verbindung nach Anspruch 1 oder 2 mit mindestens einem von der Verbindung unterschiedlichen oberflächenaktiven Mittel enthält, das aus der Gruppe ausgewählt ist, die aus nichtionischen oberflächenaktiven Mitteln, anionischen oberflächenaktiven Mitteln und kationischen oberflächenaktiven Mitteln besteht.

4. Verfahren zur Herstellung einer Polymeremulsion, welches die Polymerisation von Monomeren unter Einsatz des Emulgiermittels für die Emulsionspolymerisation nach einem der Ansprüche 1 bis 3 in einer Menge von 0,1 bis 20 Gew.-%, bezogen auf das Gewicht der Gesamtmonomeren, in einem wässrigen Medium oder das Zugeben des Emulgiermittels für die Emulsionspolymerisation zu einem Polymer nach der Polymerisation der Monomeren umfasst.

5. Polymeremulsion, erhalten durch das Verfahren zur Herstellung einer Polymeremulsion nach Anspruch 4.

## Revendications

1. Emulsifiant pour polymérisation en émulsion, comprenant un composé représenté par la formule générale (1) suivante : 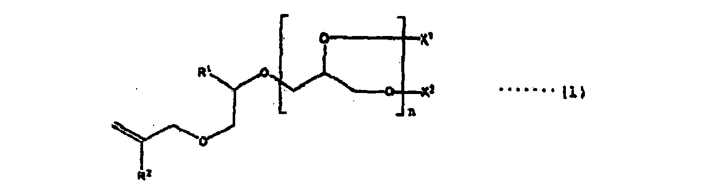 dans laquelle R¹ représente un substituant représenté par R ou -CH₂-O-R où R est choisi dans le groupe constitué par décyle, décyle secondaire, undécyle, undécyle secondaire, dodécyle, dodécyle secondaire, tridécyle, isotridécyle, tridécyle secondaire, tétradécyle, tétradécyle secondaire, hexadécyle, hexadécyle secondaire, 2-butyloctyle, 2-butyldécyle, 2-hexyloctyle, 2-hexyldécyle et phényle styréné, et de plus -O-R peut être un résidu d'un alcool choisi dans le groupe constitué par l'alcool laurylique, l'alcool myristylique, l'alcool cétylique, l'isodécanol, l'isoundécanol, l'isododécanol, l'isotridécanol, le 2-propyl-1-octanol, le 2-propyl-1-heptanol, le 4-méthyl-2-propyl-1-hexanol et le 2-propyl-5-méthyl-1-hexanol ; R² représente un atome d'hydrogène ou un groupe méthyle ; n représente de 1 à 200 ; X¹ et X² représentent chacun un atome d'hydrogène, un groupe hydrocarboné ou un groupe hydrophile anionique.

2. Emulsifiant pour polymérisation en émulsion selon la revendication 1, dans lequel la formule générale (1) a au moins l'un des groupes hydrophiles anioniques représentés par les formules générales (2) à (6) suivantes en tant que groupe hydrophile anionique : 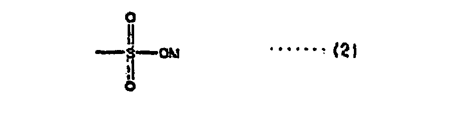 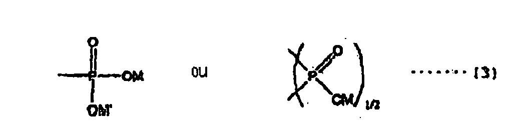 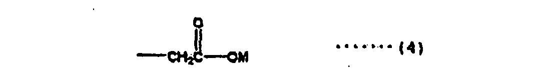 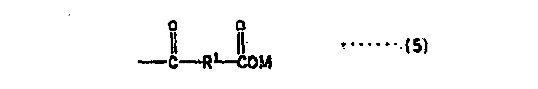 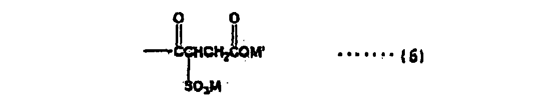 où R³ représente un résidu dans lequel un groupe carboxyle est éliminé à partir d'un acide dibasique ; M et M', qui peuvent être identiques ou différents, représentent chacun un atome d'hydrogène, un atome métallique, un ammonium ou un groupe hydrocarboné.

3. Emulsifiant pour polymérisation en émulsion, comprenant un mélange du composé selon la revendication 1 ou 2 avec au moins un agent tensioactif choisi dans le groupe constitué par les agents tensioactifs non ioniques, les agents tensioactifs anioniques et les agents tensioactifs cationiques, autre que le composé.

4. Procédé de production d'une émulsion de polymère, qui comprend la polymérisation de monomères en utilisant l'émulsifiant pour polymérisation en émulsion selon l'une quelconque des revendications 1 à 3 en une quantité de 0,1 à 20 % en poids par rapport au poids de la totalité des monomères dans un milieu aqueux, ou l'addition de l'émulsifiant pour polymérisation en émulsion à un polymère après la polymérisation des monomères.

5. Emulsion de polymère obtenue par le procédé de production d'émulsion de polymère selon la revendication 4.
